# EUROPEAN PATENT APPLICATION

(11) **EP 4 376 021 A1**
(43) Date of publication of application: **29.05.2024**
(21) Application number: 23207105.0
(22) Date of filing: 31.10.2023
(51) Int. Cl.: G16H 50/30, G16H 40/67, G16H 10/60, G16H 40/20, G06F 3/048

(54) **SYSTEMS AND METHODS FOR SEPSIS ALERTS**

(30) Priority: 22.11.2022 US 202218058223
(71) Applicant: GE Precision Healthcare LLC, Waukesha, WI 53188 (US)
(72) Inventor: CHANG, Jehoshua Joon, Suwanee, 30024 (US); TERRY, Jeffrey R., Plano, 75024 (US); MARTIN, Kathleen, Pinckney, 48169 (US); RESENDIZ MORA, Angelica, New York, 10023 (US); GURRAM, Rajitha, Arlington Heights, 60004 (US); PATEL, Anish, Philadelphia, 19122 (US); NAYLOR, Jennifer, Chicago, 60601 (US); SCHULLER, Adam, Chicago, 60610 (US)
(74) Representative: Fennell, Gareth Charles

(57) **Abstract**

Various methods and systems are provided for a sepsis alert engine (142) for sepsis detection. In one example, a computing device (102) comprising a display screen (122) displays a menu listing one or more electronic medical records (EMRs) (280) of one or more patients, and additionally is configured to display a sepsis alert graphical user interface (GUI) (300) accessible from the menu while the one or more EMR systems (280) are in an un-launched state. The sepsis alert GUI (300) displays a sepsis level element (330) identifying a sepsis level for each patient displayed as well as a limited list of criteria (322) that triggered the identified sepsis level. The limited list of criteria (322) is obtained from the one or more EMRs (280) and each criterion in the limited list is selectable to launch a pop-up UI (600) with additional information relating to the identified sepsis level.

## Description

### FIELD

Embodiments of the subject matter disclosed herein relate to sepsis alerts, and more particularly to an integrated sepsis alert system.

### BACKGROUND

Sepsis is a potentially life-threatening condition that occurs when the body's response to an infection damages its own tissues. Healthcare professionals diagnose sepsis using a number of clinical factors including laboratory results, vital signs including temperature, heart rate, respiratory rate, and blood pressure, presence of a known infection, a recent invasive/surgical procedure, and patient reported symptoms. These clinical factors are monitored by healthcare professionals in the acute setting, such as emergency departments or inpatient units like intensive care units (ICUs). Early detection and treatment of sepsis is paramount to patient recovery. Clinical factors, including vital signs and laboratory results, are repeatedly updated throughout a patient's stay in a hospital in order to maintain an up-to-date status of a patient's condition.

### BRIEF DESCRIPTION

In one example, a computing device comprises a display screen, the computing device being configured to display on the display screen a menu listing one or more electronic medical records (EMRs) of one or more patients, and additionally being configured to display on the screen the sepsis alert GUI that can be reached directly from the menu. The sepsis alert GUI may display, for each patient, a sepsis level element identifying a sepsis level for that patient and a limited list of criteria that triggered the identified sepsis level. The limited list of criteria may be obtained from the one or more EMRs, each criterion in the limited list being selectable to launch a pop-up UI with additional information relating to the identified sepsis level. The sepsis alert GUI may be displayed while the one or more EMR systems are in an un-launched state.

In another example, a method for a sepsis alert system includes displaying a menu of options for retrieving data of one or more patients from a plurality of data repositories including one or more EMRs, displaying a sepsis alert GUI which displays, for each patient, a sepsis level element indicating an assigned sepsis level determined from the retrieved data from the one or more EMRs, and in response to selection of the sepsis level element, adding additional display elements to the sepsis alert GUI, the additional display elements identifying parameters from the retrieved data from the one or more EMRs that triggered the assigned sepsis level, wherein the sepsis alert GUI is displayed while the one or more EMRs are in an un-launched state.

In another example, a sepsis alert system includes one or more processors, memory storing instructions executable by the one or more processors to receive, at the sepsis alert system, a feed from an EMR database, where one or more values for each of a plurality of parameters for each of a plurality of patients are sent over the feed, output, for display on a display device, a sepsis alert GUI that includes, for one or more patients of the plurality of patients, a sepsis level element indicating an assigned sepsis level for that patient, where each sepsis level element is generated by applying a set of rules to a set of patient data obtained from the feed, and display, on the sepsis alert GUI, for a selected patient of the one or more patients, a criteria element indicating a contributing factor to the assigned sepsis level of the selected patient.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, wherein below:
FIG. 1 shows a block diagram of an example computing system for sepsis alerts.
FIG. 2 shows a block diagram of an example module architecture for sepsis alerts.
FIG. 3 shows an example sepsis alert graphical user interface (GUI) in a command center view.
FIG. 4 shows another example sepsis alert GUI in a rounding view.
FIG. 5 shows an example pop-up user interface (UI) of a sepsis level element.
FIG. 6 shows an example pop-up UI of sepsis criteria element.
FIG. 7A shows an example UI of a sepsis module configurator.
FIG. 7B shows another example UI of a sepsis module configurator.
FIG. 8 shows a table of example patient inclusion criteria.
FIG. 9 shows a table of example sepsis prioritization.
FIG. 10 shows another example sepsis alert GUI.
FIG. 11 shows an example pop-up user interface of a diagnosis mismatch flag.
FIG. 12 shows an example pop-up user interface of a snooze alert.
FIG. 13 shows another example UI of a sepsis module in a patient manager.
FIG. 14 shows a flowchart illustrating a method for triggering and displaying a sepsis level alert.
FIG. 15 shows a flowchart illustrating a method for viewing and modifying a sepsis alert GUI.
FIG. 16 shows a flowchart illustrating a method for snoozing and unsnoozing an element in a sepsis alert GUI.

### DETAILED DESCRIPTION

The following description relates to various embodiments of a sepsis alert system. In particular, systems and methods for a sepsis alert system are provided. Hospitals and other clinical facilities may provide computing systems with graphical user interfaces (GUIs) for displaying patient information to healthcare providers and other users. In this way, a healthcare provider may view the most up-to-date patient information and retrieve data from electronic health records (EHRs), imaging results, laboratory results, and so on. Further, alerts may be automatically and/or manually generated to indicate status(es) of a patient. Such alerts may become unwieldy or less useful in an environment such as an intensive care unit (ICU). For example, patients being treated in an ICU may each be associated with multiple different alerts of different priorities and assigned to different healthcare providers, and various alerts may be triggered by different computing systems and presented to healthcare providers via different GUIs.

Sepsis, as a potentially life-threatening condition, necessitates early detection and treatment for patient recovery. Clinical data, such as laboratory results, including markers for systemic inflammatory response syndrome (SIRS) and organ dysfunction, lactate levels, and blood cultures, suspected or known sources of infection, vital signs, including blood pressure, heart rate, breathing rate, and temperature, and algorithmic scores like a modified early warning score (MEWS) and a penetration/aspiration score (PAS), are used to diagnose and rule out sepsis. With multiple GUIs providing information of a vast number of patients to multiple different healthcare providers, detection of sepsis may be delayed. Without a single GUI to provide information regarding a sepsis concern or diagnosis, healthcare providers may resort to manually sorting lab results, vitals, and other clinical information in order to diagnose sepsis, which may inordinately take time and delay indicated treatment for patients.

The methods and systems provided herein detail a sepsis alert system in a single graphical user interface that enables users to easily review sepsis alerts, view the criteria triggering the alerts, adjust the status of the alerts, snooze or dismiss the alerts, escalate the alerts, configure thresholds for sepsis criteria, configure weights of criteria for prioritization, and configure the scope of different sepsis criteria. These interactions with the alerts may be applied to the display of such alerts to other users. In this way, alerts may be effectively managed so that healthcare providers may be able to detect sepsis earlier and consequently start treatment earlier.

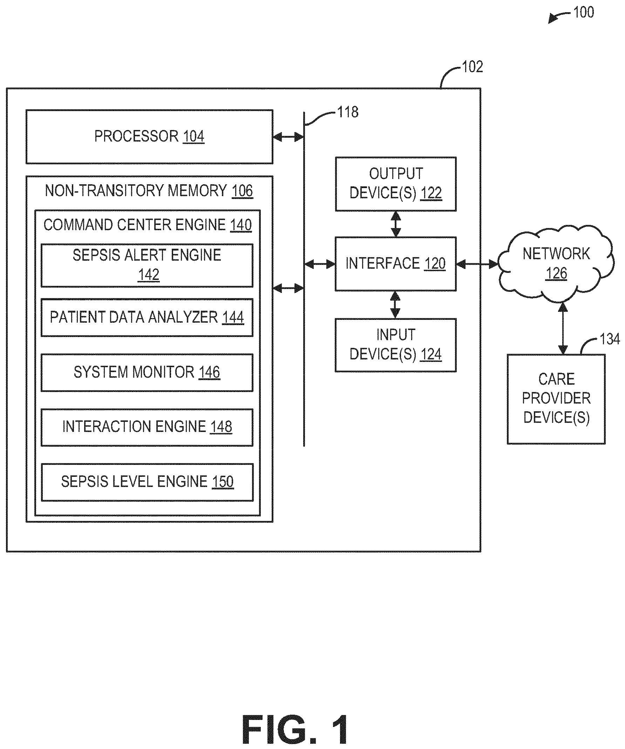

Referring now to FIG. 1, an example of a computing system 100 is shown. Computing system 100 comprises a computing device 102 which may comprise, as illustrative and non-limiting examples, a server, a personal computer, a workstation, a mobile device (e.g., a cellular phone, a smart phone, a computing tablet, and so on), or any other type of computing device.

The computing device 102 includes a processor 104 which may be configured to execute machine-readable instructions stored in non-transitory memory 106. Processor 104 may be single core or multi-core, and the programs executed thereon may be configured for parallel or distributed processing. In some embodiments, the processor 104 may optionally include individual hardware components that are distributed throughout two or more devices, which may be remotely located and/or configured for coordinated processing. In some embodiments, one or more aspects of the processor 104 may be virtualized and executed by remotely-accessible networked computing devices configured in a cloud computing configuration. The computing device 102 further includes non-transitory memory 106. It should be appreciated that the computing device 102 may include additional memory devices, including volatile memory, mass storage, local memory, and so on.

In some examples, the non-transitory memory 106 may include components disposed at two or more devices, which may be remotely located and/or configured for coordinated processing. In some embodiments, one or more aspects of the non-transitory memory 106 may include remotely-accessible networked storage devices configured in a cloud computing configuration. The processor 104 and the non-transitory memory 106 may be coupled, for example, via a communications bus 118.

The computing device 102 may further include an interface 120 communicatively coupled to the processor 104 and the non-transitory memory 106 via the communications bus 118. The interface 120 may be implemented by one or more of any type of interface standard, such as an Ethernet interface, a universal serial bus (USB), a BLLTETOOTH interface, a near field communication (NFC) interface, and/or a PCI express interface.

The computing device 102 may further include one or more output device(s) 122 communicatively coupled to the processor 104 and the non-transitory memory 106 via the interface 120. The output device(s) 122 may comprise, for example, one or more display devices. Such a display device may include one or more display devices utilizing virtually any type of technology (e.g., a light emitting diode (LED), an organic light emitting diode (OLED), a liquid crystal display (LCD), a cathode ray tube (CRT) display, an in-place switching (IPS) display, a touchscreen, and so on). In some examples, output device 122 may comprise a computer monitor configured to display medical information of various types and styles. Output device(s) 122 may be combined with processor 104, non-transitory memory 106, and/or user input device(s) 124 in a shared enclosure, or may be a peripheral display device and may comprise a monitor, touchscreen, projector, or other output device known in the art, which may enable a user to view decision support output (e.g., alerts) according to one or more examples of the current disclosure, and/or interact with various data stored in non-transitory memory 106.

The computing device 102 may further include one or more user input device(s) 124 coupled to the processor 104 and the non-transitory memory 106 via the interface 120. A user input device 124 may comprise, for example, one or more of a touchscreen, a keyboard, a mouse, a trackpad, a motion sensing camera, a microphone, or other device configured to enable a user to interact with and manipulate data within computing device 102.

The interface 120 may further include a communication device such as a transmitter, a receiver, a transceiver, a modem, a residential gateway, a wireless access point, and/or a network interface to facilitate exchange of data with external machines (e.g., computing devices of any kind) via a network 126. For example, the communication may be via, for example, an Ethernet connection, a digital subscriber line (DSL) connection, a telephone line connection, a coaxial cable system, a satellite system, a line-of-site wireless system, a cellular telephone system, and so on. As a non-limiting example, FIG. 1 shows one or more care provider devices 134 that may be communicatively coupled to computing device 102. Each care provider device may include a processor, memory, communication module, user input device, display (e.g., screen or monitor), and/or other subsystems (similar to the processor, memory, communication module, user input device, and output device of computing device 102) and may be in the form of a desktop computing device, a laptop computing device, a tablet, a smart phone, or other device. Each care provider device may be adapted to send and receive encrypted data and display medical information, including medical images in a suitable format such as digital imaging and communications in medicine (DICOM) or other standards. As will be explained in more detail below, the care provider devices may display graphical user interfaces described herein (including alerts generated by a sepsis alert engine 142) and may facilitate user interaction with the graphical user interfaces (e.g., to snooze, dismiss, or perform other actions for an alert).

The command center engine 140 may comprise a sepsis alert system configured to enable evolving statuses and decision support associated with sepsis alerts. The command center engine 140 includes, as an illustrative and non-limiting example, a sepsis alert engine 142, a patient data analyzer 144, a system monitor 146, an interaction engine 148, and a sepsis level engine 150. The patient data analyzer 144 processes available patient data for patients of a healthcare facility to understand the patient population. For example, the patient data analyzer 144 tracks patients, associates them with sepsis criteria and sepsis levels. The sepsis level engine 150 integrates data from the patient data analyzer 144 in order to determine which sepsis criteria a patient may or may not meet, to assign an appropriate sepsis level to a patient, and to update a sepsis level of a patient. The system monitor 146 monitors a total resource load on the healthcare facility from patients and pathways and draws information from the patient data analyzer 144 and the sepsis level engine 150 to monitor patient progress with regard to sepsis level status, evaluate which patients should be assigned which sepsis level, predict likely outcomes for patients with particular sepsis levels, and so on.

The interaction engine 148 enables one or more users and/or external systems (e.g., an electronic medical record system, a picture archiving and communication system, a radiology desktop, an imaging workstation, an administrative workstation, and so on) to interact with patient and/or sepsis level information via the system monitor 146, sepsis level engine 150, patient data analyzer 144, and the sepsis alert engine 142. For example, changes can be made to patient records, prescriptions, associated protocols/tasks, and so on, via the interaction engine 148. A display driver may generate a graphical user interface to display information on an output device 122 such as a display screen, for example, and facilitate interaction with the interaction engine 148.

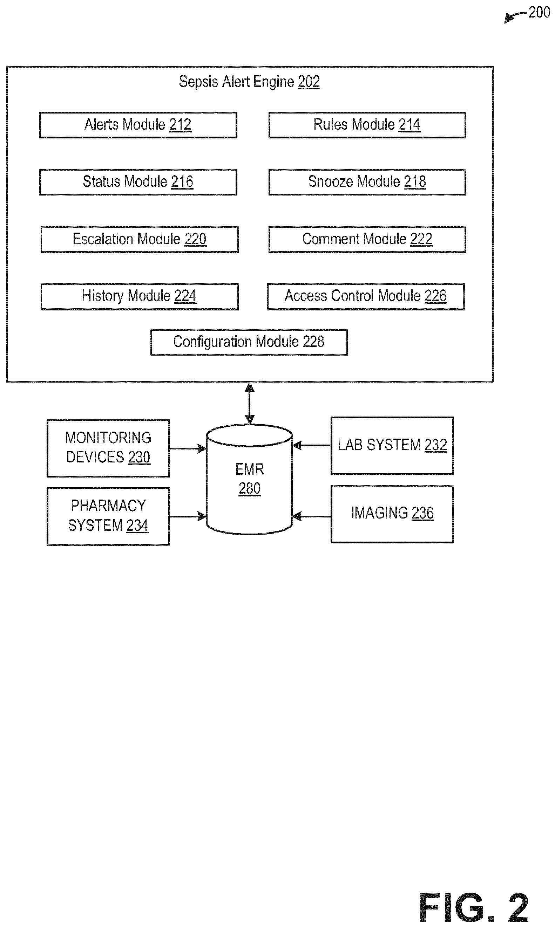

The sepsis alert engine 142 integrates into any graphical user interface or graphical user interface module (e.g., a tile) for enabling sepsis alerts. As described further herein with regard to FIG. 2, the sepsis alert engine 142 provides alerts relating to a patient responsive to electronic medical record (EMR) signals and/or manual input, and further enables management of the alerts by selectively allowing a user to change a status of the alert, snooze or dismiss the alert, escalate the alert, comment on the alert, view history of the alert and actions relating to the alert, delete the alert, and so on.

The sepsis level engine 150 looks at a series of alerts from the sepsis alert engine 142, for example, for a patient and determines which sepsis criteria the patient has met. For example, the patient may have a most recent lactic acid level of greater than 4, which, on its own or in combination with other criteria the patient has also met, may trigger a particular sepsis level. As an illustrative example, the sepsis level engine 150 looks at sepsis criteria alerts regarding particular lab values, algorithmic scores, or demographics like a recent procedure, among other parameters. The sepsis level engine 150 includes a configuration table that includes all possible combinations of such criteria alerts and maps each combination to a specific sepsis level of the patient. Determination and identification of a sepsis level based on a plurality of patient parameters may stem from the configuration table and rule-based inputs from the electronic medical record database. As an example, a patient exhibiting a combination of identified parameters including a lactic acid greater than 4 and refractor hypotension retrieved from an EMR may qualify the patient for a "Sepsis Concern" sepsis level based on the configuration table. An assigned sepsis level may be changed based on new patient parameters acquired from one or more EMR systems. As an illustrative example, the sepsis level engine 150 may evaluate a combination of criteria (e.g., sepsis criteria) at multiple intervals of time as new patient data becomes available, and determines that the patient, who initially may have been classified as a "Sepsis Concern," may be upgraded to a higher sepsis level, such as "Severe Sepsis" (the meaning of such levels will be further described below).

It should be understood that computing system 100 shown in FIG. 1 is illustrative and non-limiting, and that another appropriate computing system 100 may include more, fewer, or different components.

FIG. 2 shows a block diagram of an example sepsis alert system 200 for sepsis alert management. The sepsis alert system 200 includes a sepsis alert engine 202 which may comprise the sepsis alert engine 142 of the command center engine 140, as an illustrative and non-limiting example. The sepsis alert engine 202 may include an alerts module 212, a rules module 214, a status module 216, a snooze module 218, an escalation module 220, a comment module 222, a history module 224, an access control module 226, and a configuration module 228. The sepsis alert engine 202 may generate sepsis alert elements to be displayed within a user interface (UI) as a tile.

The alerts module 212 generates alerts for patients responsive to automatic triggers (e.g., from EMR signals) and/or manual commands. Alerts may be displayed in the form of widget elements, such as sepsis level elements. For example, the rules module 214 stores rules for evaluating EMR signals to determine alerts based on the EMR signals, determine if alerts are resolved based on EMR signals, and/or determine if conflicts or mismatches exist between alerts. The status module 216 manages a status of an alert (e.g., complete, in progress, incomplete, ruled out, or resolved). The status module 216 may, in some examples, further manage a priority of the alert. The snooze module 218 enables a user to snooze or temporarily dismiss an alert. In some examples, an alert may be snoozed or dismissed for a set amount of time. In other examples, a snooze may be removed from an alert if additional or new data pertaining to a patient is acquired from one or more EMR systems and the sepsis alert engine 202 obtains new sepsis criteria that either reactivate a snoozed sepsis level element or upgrade a sepsis level to a higher weighted sepsis level. The escalation module 220 enables a user to escalate an alert to one or more users or departments. The comment module 222 enables a user to provide a comment on an alert.

The sepsis alert engine 202 operates globally across all users of the command center engine/sepsis alert engine. For example, if one user snoozes an alert, then all users will see that alert as snoozed. This applies for all actions taken by the command center engine. In some examples, the command center engine may have an edit feature provided via access control module 226 that enables permissions/actions for each alert to be controlled at the user, group, or core level. For example, permissions for any action may be managed at the user group level so that a local team can adjust a specific alert or alert type to meet their specific implementation.

The access control module 226 controls access to different functions of the sepsis alert engine 202. Access to alert manager functions may be defined at a tile by tile level. The level of access may be at the following levels: User Can Snooze (Y/N), User Can Dismiss (Y/N), User Can Reactivate (Y/N), User Can Manage (Y/N), User Can Mark In Progress (Y/N), User Can Assign (Anyone, Self Only, No), User Can Configure Tile (Y/N), User Can Mark Priority (Y/N).

The history module 224 logs any and all action taken by a user regarding an alert. This history may be maintained such that it can be displayed on the tile and audited afterwards by authorized users. The information includes user ID, time of event, action taken, and any reason comments associated.

The configuration module 228 allows for customization of prioritization and weighting of sepsis criteria and sepsis levels, amongst others. Each sepsis criterion may be given a weight (e.g., a number). The higher weighted criteria may trigger higher priority than lower weighted criteria. The combination of the criteria that a patient meets may be used by the sepsis level engine 150 in order to determine the appropriate sepsis level for the patient. A patient with higher weighted, and therefore higher priority, criteria, may be assigned a more severe (e.g., higher weighted) sepsis level.

The configuration module 228 may also allow for customization of a scope of a sepsis criterion that contain parameters therein. For example, SIRS is a sepsis criterion and certain parameters, like vital signs or lab values, may be met in order for SIRS to be met as a sepsis criterion. The scope of each criterion is configurable within the configuration module 228. For example, the scope includes the timeframe from which each parameter that may be met can be taken from (e.g., a window in time in which the parameter is analyzed to determine if the parameter meets a specific condition that may indicate the criterion has been met). As an example, the sepsis alert system 200 may be configured to look at a plurality of parameters that indicate a patient meets a SIRS criterion from the most recent 6 hour period, however the sepsis alert system 200 may be configured to look at a plurality of parameters that indicate a patient meets an Organ Dysfunction criterion from a most recent patient data value of a particular type of lab value (e.g., can look at the most recent white blood cell count and the most recent creatinine level, but not two values of the same type) from the most recent twelve hour period.

The configuration module 228 may further allow for customization of thresholds of criteria. As an example, patient temperature may be a sepsis criterion. Patient temperature thresholds may include a high threshold set to 101°F and a low threshold set to 96.8°F. A patient with a temperature value outside of the threshold range of 96.8°F to 101 °F may meet a temperature sepsis criterion within the sepsis alert engine 202. A temperature value that is outside this range, may be displayed as a sepsis criteria element for a patient with a sepsis level element. A temperature value that is within the threshold range may not be displayed as a sepsis criteria element for a patient with a sepsis level element.

As explained above, the alerts module 212 generates alerts for patients responsive to automatic triggers, which may include triggers from EMR signals. As such, the sepsis alert engine 202 is communicatively coupled to an EMR database 280. EMR database 280 may store EMRs for a plurality of patients. An EMR for a patient may include patient demographic information, family medical history, past medical history, lifestyle information, comorbidities (e.g., preexisting medical conditions), current medications, allergies, surgical history, past medical screenings and procedures, past hospitalizations and visits, etc. EMR database 280 may be an external database accessible via a secured hospital interface, or EMR database 280 may be a local database (e.g., housed on a device of the hospital). EMR database 280 may be a database stored in a mass storage device configured to communicate with secure channels (e.g., HTTPS and TLS), and store data in encrypted form. Further, the EMR database is configured to control access to patient electronic medical records such that only authorized healthcare providers may edit and access the electronic medical records.

Further, sepsis alert engine 202 may communicate with other data sources that may supply triggers for generating alerts, such as lab system 232, pharmacy system 234, one or more monitoring devices 230, and imaging services 236. Other systems may be communicatively coupled to the sepsis alert engine 202 (directly and/or through EMR database), such as a computerized provider order entry (CPOE) system (which may track/manage provider orders such as treatments, tests, hospital ward/unit assignment changes, and the like) and the like.

The lab system 232 may include one or more computing devices associated with an on-site or off-site laboratory that performs lab tests on patient specimens. The one or more computing devices may include resources (e.g., memory and processors) allocated to store and execute a laboratory information system (LIS). The LIS may manage various aspects of the laboratory procedures, such as managing/assisting with tagging of incoming specimens (e.g., with patient and care provider information, test(s) to be conducted on the specimen, and so forth), tracking specimens (e.g., in storage, being processed), generating reports of test results, and the like. Accordingly, the LIS may interface directly with various laboratory equipment, such as mass spectrometers, chromatographers, analyzers, etc., and thus may have knowledge of which specimens are currently being tested, the results of such tests, and the performance status of the various pieces of equipment. The lab system 232 may send lab results to EMR database 280, and the lab results may be included in the EMR signals. Additionally or alternatively, the lab system 232 may send lab results directly to sepsis alert engine 202, at least in some examples, and thus the sepsis alert engine 202 may receive lab signals from which alerts may be generated.

The pharmacy system 234 may include one or more computing devices associated with an on-site or off-site pharmacy that fills prescriptions as ordered by care provider(s). The one or more computing devices of the pharmacy system may include resources (e.g., memory and processors) allocated to receive prescription requests and communicate the requests with pharmacy staff, track prescription fill status, notify an ordering care provider when a prescription is available, and so forth. The pharmacy system 234 may send prescription notifications to EMR database 280, and the prescription notifications may be included in the EMR signals. Additionally or alternatively, the pharmacy system 234 may send notifications regarding prescriptions directly to sepsis alert engine 202, at least in some examples, and thus the sepsis alert engine 202 may receive pharmacy signals from which alerts may be generated.

The monitoring devices 230 may include traditional medical devices monitoring respective patients, such as pulse oximeters, heart rate monitors, blood glucose monitors, and electrocardiograms (ECGs), as well as microphones, cameras, and other devices. The monitoring devices 230 may send output directly to the sepsis alert engine 202 and/or may send output to the EMR database 280. The imaging services 236 may include a radiology information system (RIS), a picture archive and communication system (PACS), and/or other computing devices associated with diagnostic imaging. The computing devices comprising the imaging services 236 may manage scheduling of diagnostic imaging exams, recommend and execute scanning protocols to perform diagnostic imaging exams, process imaging data to generate images, save diagnostic images in memory, etc. The imaging services 236 may send information/signals regarding diagnostic imaging to the EMR database 280 and/or directly to the sepsis alert engine 202.

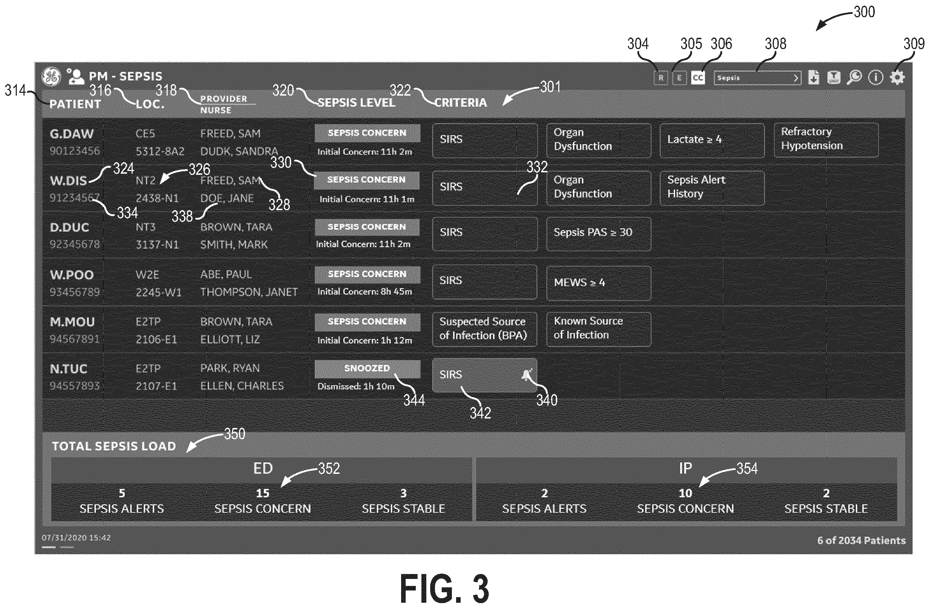

Turning to FIG. 3, an example of a sepsis alert graphical user interface (GUI) 300 is shown of a sepsis alert engine, such as the sepsis alert engine 202 of FIG. 2. Sepsis alert GUI 300 may be displayed on a display device (e.g., a display device of a care provider device 134). Specifically, sepsis alert GUI 300 may be displayed to a care provider when the care provider uses the sepsis alert engine to view patient information for one or more patients of a medical facility that have current sepsis alerts viewable in the sepsis module.

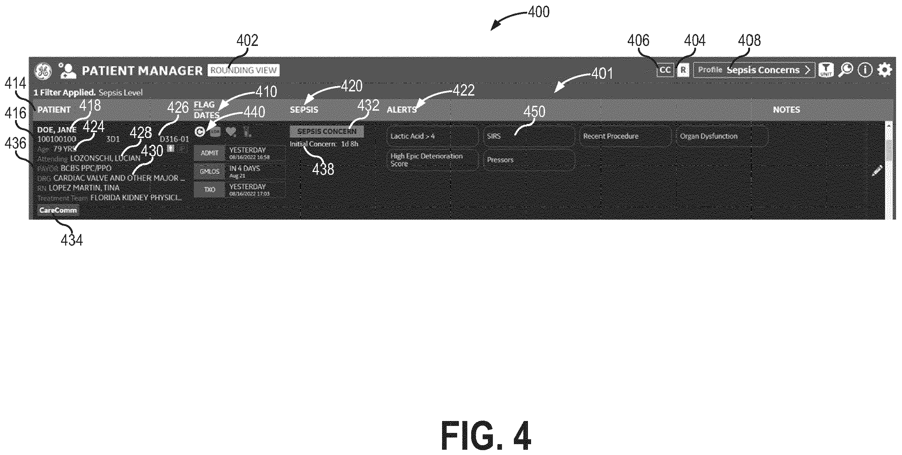

As depicted in FIG. 3, sepsis alert GUI 300 is in a command center view of the sepsis alert engine. The sepsis alert GUI 300 may be displayed in different alternative views. In some examples, the sepsis alert GUI 300 may be displayed either in an enhanced or rounding view (an example of a rounding view is shown in FIG. 4), where information is displayed in a fixed format that is not customizable by the user, or in a condensed or command center view, where less information is displayed in a variable format that is customizable by the user. The user may customize the condensed view by hiding one or more columns, rows, sections, or elements of the enhanced view. Each individual user may select a desired view and/or switch between desired views by selecting one or more of the controls of the sepsis alert GUI 300, such as a rounding view toggle switch 304, enhanced view toggle switch 305, or a command center view toggle switch 306. In the current depiction shown in FIG. 3, the command center view toggle switch 306 is selected, indicating that the sepsis alert GUI 300 is in a command center view, as previously stated.

The sepsis alert GUI 300 may include a profile element 308. Via the profile element 308, the user may select a desired profile for how information is displayed via sepsis alert GUI 300. For example, the selected profile may highlight or emphasize a sepsis level (e.g., "Sepsis Concern" vs "Severe Diagnosis") and thus sort the information displayed by the sepsis alert GUI 300 based on the selected profile (e.g., displaying patients with sepsis diagnoses or sepsis concerns above patients without sepsis diagnoses or sepsis concerns). Other profiles may highlight or emphasize other factors, such as sepsis criteria or treatment alerts. Additionally, in some examples, one or more filters may be applied to the sepsis alert GUI 300 to filer patient data shown in the sepsis alert GUI 300. The one or more filters may be selected, for example, via controls accessible via a settings button 309.

As one example, a user may want to look at all patients listed as a "Sepsis Concern" level and thus may want to filter out patients who have a "Ruled Out" level (where sepsis has been ruled out, at least currently) or a "Resolved Dx" level (where a patient had a sepsis alert but the condition has resolved), for example. The user may select to filter patients by "Profile: Sepsis Concerns", which may result in sepsis alert GUI 300 displaying only patients who have been assigned the sepsis level of "Sepsis Concern".

In the example shown in FIG. 3, sepsis levels, sepsis criteria, and health care teams may be displayed in columns of sepsis alert GUI 300, where each row corresponds to a patient within the medical facility. The sepsis levels and other information may be displayed in columns of sepsis alert GUI 300. In other examples, a layout of the information may be different. For example, in other embodiments, each patient within the sepsis module may be displayed in a separate column, and the sepsis levels and other information may be displayed in rows of sepsis alert GUI 300. When the sepsis alert engine is in a command center view, as is depicted in sepsis alert GUI 300, the columns displayed in the display panel 301 may be configured by the user. It should be appreciated that the layout of the elements of a sepsis alert GUI may vary in different examples, and the elements may appear in different visual configurations without departing from the scope of this disclosure. Additionally, not all of the elements shown in sepsis alert GUI 300 may be included in an example, and some examples may include a greater or lesser number of elements.

Sepsis alert GUI 300 may include a patient information column 314. In some examples, sepsis alert GUI 300 may display multiple patients at one time and the patient information column 314 relating to each patient may provide less identifying information than a graphical user interface configured to display only one patient, in which case a full patient data panel may be displayed (as is shown later with reference to FIG. 4). In the example shown in FIG. 3, the patient information column 314 of sepsis alert GUI 300 includes a patient name 324 and a patient identification number 334. In other examples, less, additional, or other information may be displayed in the patient information column 314.

In some examples, the sepsis alert GUI 300 may display a location code column 316 which may indicate a current location of a patient in the hospital or hospital system. A location code, such as location code 326, may indicate a hospital unit, room, and bed. In some examples, the sepsis alert GUI 300 may display a healthcare team column 318. Each patient listed in a row of the sepsis alert GUI 300 may be assigned to one or more providers and one or more nurses. For example, provider 328 and nurse 338are displayed within the healthcare team column 318 for a patient with patient name 324.

Sepsis alert GUI 300 may further include a sepsis level column 320, which may include a sepsis level element for each patient row displayed in sepsis alert GUI 300 (e.g., visually indicating an assigned sepsis level, such as "Sepsis Concern"), to alert a user to a potential or confirmed diagnosis of sepsis. In some examples, a limited list of possible sepsis levels may be pre-defined, where the sepsis level element displayed for a particular patient (e.g., sepsis level element 330) may be a most appropriate sepsis level identified for the selected patient from the limited list of possible sepsis levels. The sepsis alert engine may assign the most appropriate sepsis level to each patient in the medical facility based on applying a set of rules corresponding to patient data and parameters according to various criteria retrieved from a plurality of databases, as will be later described.

For example, in some embodiments, the limited list of possible sepsis level elements may include a "Sepsis Alert" level which may indicate that a patient has a sepsis alert order but does not have a diagnosis of sepsis; a "Sepsis Dx" level which may indicate that a patient has a sepsis alert order with a diagnosis; a "Severe Dx" level which may indicate that a patient has a sepsis alert order with a severe sepsis diagnosis; a "Shock Dx" level which may indicate that a patient has a sepsis alert order with a septic shock diagnosis; a "Sepsis Concern" level which may indicate that a patient meets sepsis criteria but does not have a sepsis alert order; a "Ruled Out" level which may indicate that a patient has a sepsis alert order with a ruled out diagnosis; a "Resolved" level which may indicate that a patient has a sepsis alert order with a resolved diagnosis; an "Alert >72 Hrs" level which may indicate that a patient has a sepsis alert order but with no sepsis alert over a duration of 72 hours, a "Sepsis Stable" level which may indicate that a patient met sepsis criteria but has been stable for a defined period of time, and a "Snoozed" level which may indicate that a patient met sepsis criteria but a user had snoozed the alert (e.g., after reviewing the criteria). In other examples, fewer, additional, or other sepsis levels may be used.

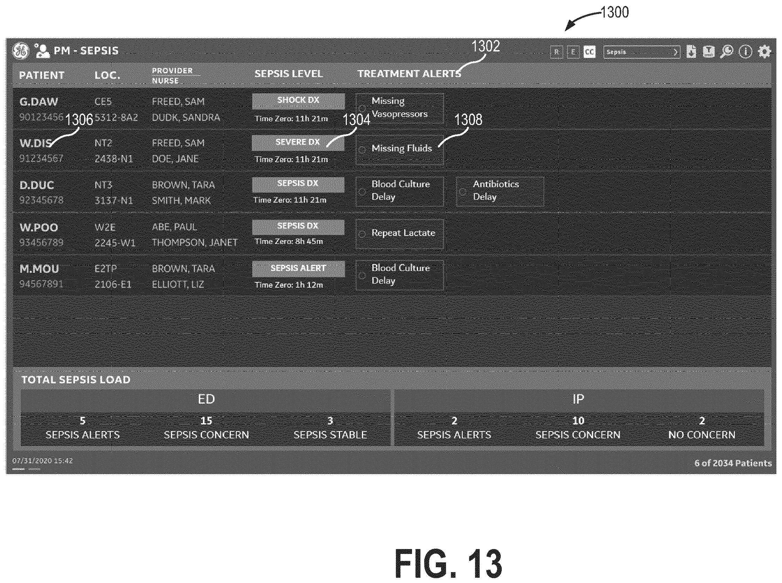

Sepsis alert GUI 300 may further include sepsis criteria column 322, which may show one or more sepsis criteria elements from a limited list of possible sepsis criteria. A sepsis criteria element that is displayed for a patient indicates a contributing factor to an assigned sepsis level for that patient. The sepsis criteria elements may be the causal criteria that were met to trigger the sepsis alert engine to display the assigned sepsis level in the sepsis level column 320. For example, a sepsis criteria element 332 may be included in a row of sepsis alert GUI 300 corresponding to a patient, indicating that the patient is exhibiting SIRS, which is a contributing factor to the assigned sepsis level for the patient. Each sepsis criterion that is met by a patient may be displayed in a row of the sepsis criteria column 322 corresponding to that patient. The one or more sepsis criteria for each patient may be triggered based on EMR data, as determined by the sepsis alert engine of FIG. 2. For example, the sepsis alert engine may generate an alert element for a patient indicating that the patient's lactic acid level is greater than 4, that the patient meets criteria for SIRS, that the patient has a known source of infection, and/or that the patient meets other suitable causal criteria, which may be displayed as sepsis criteria elements on sepsis alert GUI 300. In other examples of a sepsis alert GUI, treatment alerts may be displayed as a column, with a row of treatment alerts corresponding to a patient instead of the sepsis criteria column, as will be described later with reference to FIG. 13.

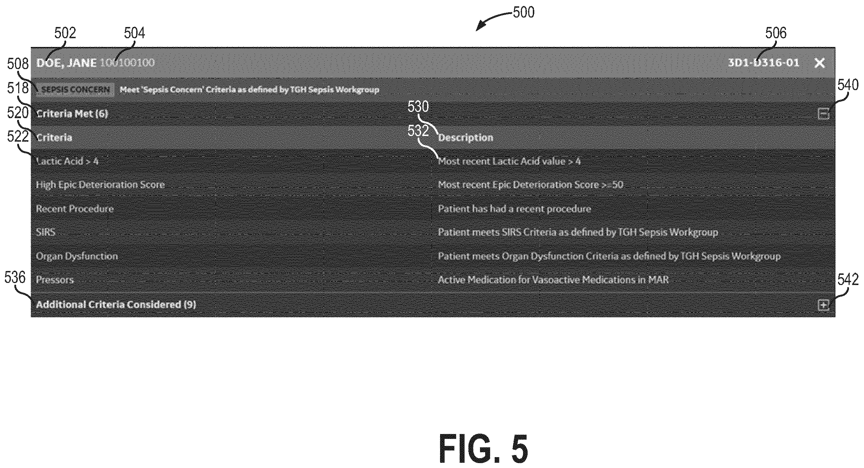

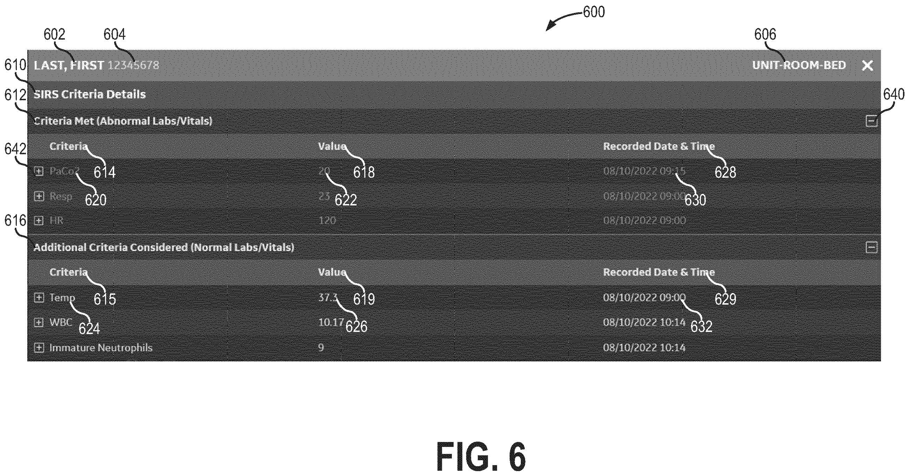

In some examples, the one or more sepsis criteria elements may be controls that may be selected or hovered over to display additional information in additional display elements in an additional display panel (e.g., a pop-up UI). When a sepsis criteria element is selected, additional information regarding the sepsis criterion with specific reference to the patient of concern may be displayed via an additional (e.g., pop-up) display panel. An example of such an additional display panel is described later with regard to FIG. 6. Similarly, each sepsis level element (such as sepsis level element 330) may be a selectable element that may be selected or hovered over to display additional information. When a sepsis level element is selected, additional information including the sepsis criteria that were met in order to trigger the sepsis level alert may be displayed via an additional display panel. Examples of such additional display panels in the form of pop-up UIs are described later with regard to FIGS. 5, 6, 11, and 12.

In some examples, one or more sepsis criteria elements may include a snooze icon during conditions in which a user has snoozed the sepsis level element for a patient. For example, FIG. 3 shows a snooze icon 340 displayed within a sepsis criteria element 342 for a patient. The snooze icon 340 may be displayed within the element of the sepsis criteria that the patient had met. In such an example when there is a snooze icon within the sepsis criteria element, the corresponding sepsis level element may be displayed as snoozed. For example, a sepsis level element 344 for the patient is displayed as snoozed. As is further described below, the snooze icon 340 may be removed by the sepsis alert engine if the patient later meets additional sepsis criteria or the system-determined sepsis level is changed based on new retrieved data from one or more EMR systems, which may unsnooze the sepsis level element 344 of the patient. Thus, unsnoozing the sepsis level element 344 may remove the snooze icon 340 from the sepsis level element 344.

In some examples, the sepsis alert GUI 300 may further display a sepsis load window 350. The sepsis load window 350 may display an emergency department sepsis load 352 and an inpatient sepsis load 354. Each of the emergency department sepsis load 352 and the inpatient sepsis load 354 may display the number of patients in the respective unit with "Sepsis Alert," "Sepsis Concern," and "Sepsis Stable." Displaying a breakdown of the number of patients in each category may allow a care provider using the sepsis alert GUI 300 to easily visualize how many patients in the hospital or hospital system are being monitored or treated for sepsis.

In some examples, the sepsis alert GUI 300, or other similar GUIs, may be launched/displayed in response to a request by a care provider when the care provider wishes to view patient information for one or more patients currently being monitored/tracked by the sepsis alert engine. Additionally or alternatively, the sepsis alert GUI 300 may be continuously running, and elements of the patient data may be periodically updated automatically by the sepsis alert engine. For example, a care provider may view sepsis alert GUI 300 to identify which patients of a plurality of patients have a snoozed alert at a first time. At the first time, the care provider may see that one or more patients have a snoozed alert. The care provider may cease viewing of the UI to attend to patients, and return later to view the UI, which may still be running, to view the alert status of the plurality of patients at a second time. In between the first and second time, elements of patient data may change, for example, due to more recent data (e.g., laboratory results, vital signs, etc.) being recorded in the EMR. As a result of the more recent data being recorded, at the second time, the care provider may see that a patient who previously had a snoozed alert now displays additional sepsis criteria elements indicating the patient now meets additional criteria triggered by the more recent data, and thus the patient's snoozed alert may have been removed by the sepsis alert engine and the patient may now be listed with an active sepsis level element which can be reviewed or viewed (e.g., seen) by the care provider. Thus, the command center engine, through the sepsis alert GUI 300, may serve to provide continuously updated data that may aid the care provider in detecting and diagnosis sepsis.

Additionally, data retrieved, accessed, or used by the command center engine may become temporarily or periodically unavailable, for example, if a system or network coupled to the command center engine experiences a failure. In the event that data is unavailable at a time when a care provider is viewing alerts and levels via the sepsis alert GUI 300, the command center engine may provide an indication of the unavailability of the data in the sepsis alert GUI 300. For example, a sepsis criterion may be replaced with a different graphical element indicating that a corresponding criterion is pending the availability of the data (e.g., restoration of the system/network that failed).

For example, in order to apply a rule to determine whether a measured lab value of a biomarker of a patient is within a suitable range, the command center engine may attempt to retrieve a recent lab test result from the EMR. A most recent test result retrieved from the EMR may not meet a threshold date or time for being sufficiently recent. In response to the most recent test result not being sufficiently recent, the command center engine may not display a sepsis criterion alert for the patient, and may display an alternative element indicating that a sufficiently recent lab test result is unavailable. In some examples, the most recent test result may be indicated along with a date and/or time of the most recent test result. Such threshold dates and times for a particular test result may be configurable by a user (e.g., a hospital system administrator) both with regard to the scope of a set of test results (as such may be the case for the SIRS or organ dysfunction criteria) as well as upper and lower thresholds of individual criteria values.

Thus, while patient data relevant to the criterion is updated asynchronously in one or more EMR systems and/or databases coupled to the command center engine, a most recent version of the patient data may be repeatedly updated and displayed in real time in the sepsis alert GUI 300. In this way, the command center engine may provide an efficient and user-friendly way to monitor patient data relating to sepsis criterion via a single interface, without having to repeatedly access various EMR systems to ensure accuracy of the patient data and/or while the one or more EMR systems are in an un-launched state (e.g., not consuming computing and/or network resources of the healthcare system). As discussed, the sepsis alert engine may obtain information from the one or more EMR systems when the one or more EMR systems are in an unlaunched state, thus allowing for reduction of a number of times a user accesses the one or more EMR systems themselves (e.g., in a separate window). Reducing the number of times the one or more EMR systems are accessed may reduce bandwidth occupied by the one or more EMR systems and may reduce data processing demands of the computing device.

An additional advantage of using the command center engine is that errors made from inappropriately treating or working up (or failing to treat/work up) to a patient due to patient data that is out of date may be reduced. If the care provider wishes to verify an element of patient data displayed in the sepsis alert GUI 300, the care provider may launch a relevant EMR system to verify the criterion (or values particular to the criterion) from the sepsis module.

Alternatively, in various examples, the care provider may open the command center engine (e.g., to view the sepsis alert GUI 300) by first logging into a patient management system of a healthcare network, and selecting a command center/patient manager icon, tile, or similar menu listing of one or more options for retrieving patient data of the patient management system to start the command center and view the sepsis alert GUI 300 of the sepsis module. In still other examples, the command center engine may be launched from within a different computer system of a hospital, such as an EMR system, where the command center engine/patient manager may be listed as a selectable menu option within a UI of the EMR system. Further, via the sepsis alert GUI 300, the care provider may view one or more sepsis criteria generated for the patient, and initiate tasks or orders relating to the sepsis criterion.

Turning now to FIG. 4, another example of a sepsis alert GUI 400 of the sepsis alert engine 202 is shown. Sepsis alert GUI 400 may be displayed on one or more display devices, such as the same display device (e.g., a display device of a care provider device 134) as sepsis alert GUI 300 is displayed on. Sepsis alert GUI 400 may be displayed in a similar fashion as sepsis alert GUI 300, including being displayed in response to selection of a user icon or an alert icon displayed on a patient manager GUI or selection of a link in an EMR, for example.

As shown, sepsis alert GUI 400 is in a rounding view, as depicted via the view field 402. A command center toggle switch 406 and a rounding view toggle switch 404 may be alternatively selected to toggle between a command center view and a rounding view. As described previously, the command center view may display information in a variable format that is customizable by the user. The rounding view, conversely, may display information in a fixed format that is not customizable by the user.

The sepsis alert GUI 400, similar to sepsis alert GUI 300, may include a profile element 408. The profile element 408 allows the user to select a desired profile for information display via sepsis alert GUI 400 in a display panel 401. The profile element 408 depicted in the sepsis alert GUI 400 is selected to display "Profile: Sepsis Concern," and thus the sepsis alert engine may be filtering to display patients with "Sepsis Concern" sepsis levels higher on a list of patients than patients with other sepsis levels.

As opposed to sepsis alert GUI 300, which displays a plurality of patients within the sepsis module in a list, sepsis alert GUI 400 is depicted as displaying one patient. Sepsis alert GUI 400, in other examples, may display more than one patient. A patient information column 414 that is displayed by sepsis alert GUI 400, which is in a rounding view, may be more or less expansive than the patient information column 314 displayed by sepsis alert GUI 300, which is in a command center view. The patient information column 414 may include a patient name 418 (or abbreviated name) and/or an identification number 416. A location code 426 may be included in the patient information column 414, which may indicate a current location of the patient in the hospital or hospital system. The patient information column 414 may further include other general information, such as, for example, an age and/or date of birth 424 of a patient, an attending physician 428 of a patient, patient insurance information 436, a diagnosis 430 of a patient (or diagnosis relation group), and the like.

In some examples, sepsis alert GUI 400 may include a flag/dates column 410. The flag/dates column 410 may include a date of admission indicating the date in which a patient was admitted to the hospital and an estimated length of stay (e.g., a geometric mean length of stay, GMLOS). Further, in the example shown in FIG. 4, some aspects of patient information may be visualized via flags. Flags, such as flag 440, may indicate information such as whether a patient is a new admission or a readmission, whether a patient has a Line, Drain, Airway (LDA), or other patient information. The flag/dates column 410 may include less or more information in other examples.

In some examples, a care communication button 434 may be included in the patient information column 414. When selected, the care communication button 434 may generate an alert that may be sent to other care providers and/or saved as part of the patient's EMR to indicate that an ordered or commanded patient task has yet to be initiated or completed. However, in some examples, the care communication button 434 may be omitted or may trigger other types of communication or alerts.

Similar to sepsis alert GUI 300, sepsis alert GUI 400 may display both a sepsis level column 420 and a sepsis criteria alerts column 422 in the display panel 401. The sepsis level column 420 may include a sepsis level element, such as sepsis level element 432, corresponding to each patient displayed in the display panel 401, as well as a timeline of initial concern 438. The timeline of initial concern 438 may display a time from when a first alert of the sepsis level was generated, in units of days and hours. The sepsis criteria alerts column 422 may include one or more sepsis criteria elements, such as sepsis criteria element 450, in each patient row.

In some examples, sepsis level elements, such as sepsis level element 432, and sepsis criteria alert elements, such as sepsis criteria element 450, may be selectable so as to modify the sepsis alert GUI by adding additional display elements in a pop-up UI. The additional display elements in the pop-UI may display additional information relevant to the selected element. Further, in some examples, different information may be displayed when a user enters different user input such as a hover (e.g., when the user positions a cursor or other user input locator over an element but does not enter further user input) versus selecting via one or more clicks or touches of a user input device.

Turning now to FIG. 5, an example of a first pop-up LΠ 500 is shown. First pop-up LΠ 500 may be launched in response to a user selecting or hovering over a sepsis level element (e.g., sepsis level element 432) of a sepsis alert GUI, such as sepsis alert GUI 400. The first pop-up UI 500 may display additional information relating to the patient, who may be referenced by a patient name 502, identification number 504, and/or location code 506. A first sepsis level element 508 corresponding to a sepsis level element that had been selected on a previous interface display may be displayed in the first pop-up LΠ 500. The additional information displayed in the first pop-up UI 500 may relate to the first sepsis level element 508. The additional information may be displayed in one or more informational rows or panels, such as a criteria met panel 518 and an additional criteria considered panel 536. In some examples, the first pop-up UI 500 may include additional controls, such as expansion buttons, that when selected, may display additional information or add additional information to the first pop-up UI 500. In some examples, selecting an additional control may launch an additional display panel.

In some examples, both the criteria met panel 518 and the additional criteria considered panel 536 may include an expansion button. Expansion button 540, corresponding to the criteria met panel 518, is depicted in an expanded state while expansion button 542, corresponding to the additional criteria considered panel 536, is depicted in an unexpanded state. When an expansion button is in an expanded state, the corresponding panel may display each of the relevant causal criteria in a column and the descriptions of each of those criteria in another column within the panel. For example, expansion button 540 is in the expanded state as depicted in FIG. 5 and consequently the criteria met panel 518 displays a first category of criteria in a criteria column 520 and a description column 530, which include a list of the criteria that the patient met in order to trigger the sepsis level element that was selected and descriptions thereof. Expansion button 542 is in the unexpanded state and as such the additional criteria considered panel 536 does not display further information in a second category of criteria.

Criteria element 522 is an example of one of the criteria within the criteria column 520. The description column 530 displays a short description of each the corresponding criterion that were met to trigger the first sepsis level element 508. For example, criteria element 522 displays "lactic acid > 4" as the causal criterion that was met. A corresponding description 532 describes that the most recent lactic acid level, as received from one or more data repositories, was greater than 4. A lab value of a lactic acid greater than 4 may be configured as a sepsis criteria and may be included in the limited list of criteria available to be met for a particular sepsis level that may be identified to be associated with a patient.

The additional criteria considered panel 536 may contain remaining criteria that are available to be met for the selected sepsis level, but that the patient did not meet. For example, if expansion button 542 were to be switched into an expanded state, the second category of criteria in a "criteria not met" column may be displayed. A description column may also be displayed within the additional criteria considered panel 536 if the expansion button 542 were to be switched into an expanded state. Each row in the description column may correspond to a row in the criteria not met column and may provide a description for why the patient did not meet the corresponding criterion.

Turning now to FIG. 6, an example of a second pop-up UI 600 is shown. Second pop-up UI 600 may be launched in response to a user input of selecting or hovering over a sepsis criteria element in a rounding view or command center view (e.g., sepsis criteria element 450) of a GUI such as sepsis alert GUI 400. Second pop-up UI 600 may display additional information relating to the patient, including at least the selected criterion in the form of a sepsis criteria element 610. The patient may be referenced by a patient name 602, identification number 604, and/or a location code 606. The additional information displayed in the second pop-up UI 600 may relate to the sepsis criteria element 610. The additional information may be displayed in one or more informational rows or panels, such as a criteria met panel 612 and an additional criteria considered panel 616. In some examples, the second pop-up UI 600 may include additional controls, such as buttons, that when selected, may display different or additional information or modify the GUI by adding. In some examples, selecting an additional control may launch an additional display panel. Second pop-up UI 600 specifically depicts a SIRS pop-up UI and consequently, the sepsis criteria element 610 indicates a SIRS criterion that was met by the patient. Other pop-up UIs for other sepsis criteria are possible.

In some examples, both the criteria met panel 612 and the additional criteria considered panel 616 may include one or more expansion buttons, similar to the expansion buttons of first pop-up UI 500. When an expansion button, such as expansion button 640, in an expanded state, a panel, such as the criteria met panel 612, may display one or more parameters (e.g., criteria) relating to the sepsis criteria element 610 that the patient met. For example, the sepsis criteria element 610 is depicted in FIG. 6 as SIRS. SIRS may be configured in the configuration module to be defined by a plurality of parameters (e.g., a plurality of criteria or clinical indicators), including vital signs and lab values, and the patient may meet one or more of the SIRS parameters in order to meet the SIRS criteria. Thus, second pop-up UI 600 may include both the criteria met panel 612 as well as the additional criteria considered panel 616, each of which may have parameters displayed therein.

Both the criteria met panel 612 and the additional criteria considered panel 616 may include a criteria column (e.g., criteria column 614 and criteria column 615), a value column (e.g., value column 618 and value column 619), and a recorded date and time column (e.g., recorded date and time column 628 and recorded date and time column 629). This plurality of columns may visually indicate to a user which parameters a patient met within a sepsis criterion (in the example shown in FIG. 6, a SIRS criteria) and which parameters the patient did not meet, including values, descriptions, and timeframes for each parameter.

As an example, a SIRS criterion, such as sepsis criteria element 610, comprises parameters such as partial pressure of carbon dioxide (PaCO2), respiration rate, heart rate, temperature, white blood cell count (WBC), and/or immature neutrophil count that may be met in order to meet a SIRS criterion. Other parameters may be possible. As an example, in the example of second pop-up UI 600 depicted in FIG. 6, a patient has met a criteria element 620, (e.g., a partial pressure of CO2 criteria), which has a value 622 (e.g., a value of 20), and that value 622 was taken on date/time 630 (e.g., at 09:15 on 8/10/2022). Criteria element 620 is displayed in the criteria column 614 of the criteria met panel 612, the value 622 is displayed in the value column 618 of the criteria met panel 612, and the date/time 630 is displayed within the recorded date and time column 628 of the criteria met panel 612.

As another example, in the example of second pop-up UI 600 depicted in FIG. 6, a patient has not met a criteria element 624 (e.g., a temperature criteria). The criteria element 624 has a value 626 (e.g., a value of 37.3). Value 626 was acquired at date/time 632 (e.g., at 09:00 on 8/10/20220). Threshold parameters for temperature criteria may be configured within the configuration module 228. In the example just described, the patient's temperature value lies within the parameters configured that do not trigger an alert and therefore, the criteria element 624 is displayed within the additional criteria considered panel 616 (e.g., normal labs/vitals).

All of the criteria (e.g., parameters) comprised within a SIRS criterion may be listed within either the criteria met panel 612 or the additional criteria considered panel 616. Other criteria elements that a patient meets, such as Organ Dysfunction, may be displayed in another pop-up UI similar to second pop-up UI 600 in a similar fashion whereby each criteria within the Organ Dysfunction criterion may be displayed in either a criteria met panel or an additional criteria considered panel with relevant values and dates/times.

Each of the elements within the criteria column 614 may include an expansion button. For example, criteria element 620 includes an expansion button 642. Expansion button 642 is depicted in FIG. 6 in an unexpanded state. Selecting the expansion button 642 may switch the criteria element 620 to an expanded state, which may add additional information regarding the criteria element 620, including one or more values taken at different times to show a trend over time.

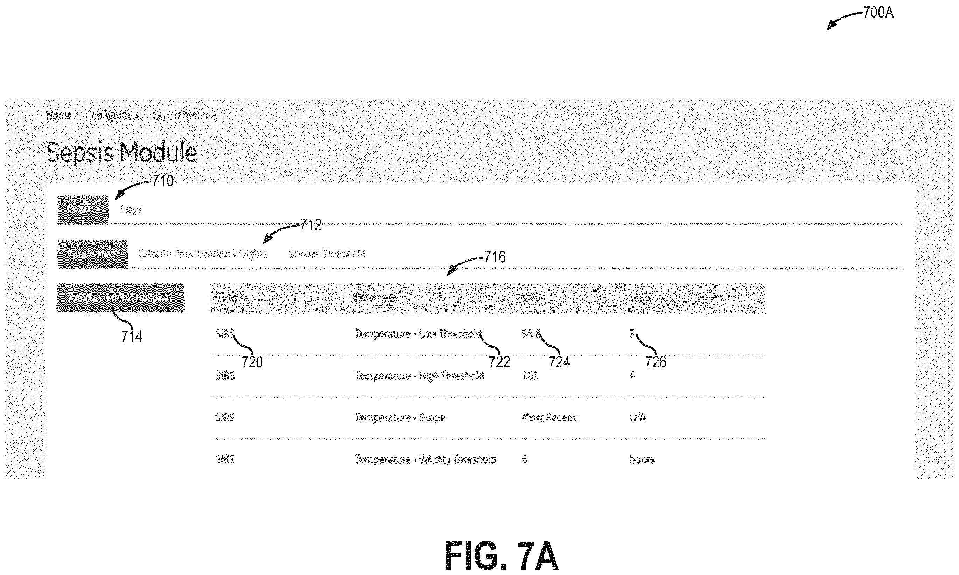

Turning now to FIG. 7A, an example of a configuration UI 700A is shown. Configuration UI 700A may be displayed on a display device viewable by a user (e.g., a facility administrator or care provider). The configuration module 228 described with reference to FIG. 2 may allow the user to configure thresholds and parameters of particular criteria, scopes of particular criteria elements, and criteria prioritization weights. FIG. 7A depicts a LΠ showing configurations of different parameters for a SIRS criterion. A configuration heading toggle 710 allows the user to switch between criteria configurations and flags configurations. A configuration type toggle 712 allows the user to choose between configurations of parameters, criteria prioritization weights, and snooze thresholds. A facility name 714 displays the hospital facility name of which the configurations displays are applied to. In some examples, there may be more than one facility name listed and the user may toggle between different hospital facilities, each of which may have different configurations for criteria elements.

FIG. 7A depicts the configuration UI 700A in a parameter configuration view, whereby a parameter element of the configuration type toggle 712 is selected. In the parameter configuration view, a configuration table 716 displays multiple columns and rows, including a criteria column, a parameter column, a value column, and a units column. Each row includes a criterion, a parameter relating to that criterion, a value of the parameter, and a unit of the value. Each parameter in the parameter column may correspond to a criterion in the criteria column. A value in the value column may correspond to a parameter of a corresponding criteria and each value may be given a corresponding unit as displayed in the units column. As an example, SIRS criteria element 720 may include parameter 722, which comprises the low threshold of temperature. Parameter 722 may be assigned a value 724. In this example the value 724 is 96.8. The value 724 may be understood with relation to a unit 726. In this example the units of the value 724 is Fahrenheit (F). An authorized user (e.g., a facility administrator) may adjust one or more of the elements/values in the configuration table 716 in order to configure thresholds for selected parameters, set timeframes/measurement instances which are to be analyzed (e.g., most recent measurement, most five recent measurements, all measurements in the last hour, etc.), and/or set validity windows for measured parameters (e.g., the most recent measurement is only valid if the measurement was taken in the prior six hours).

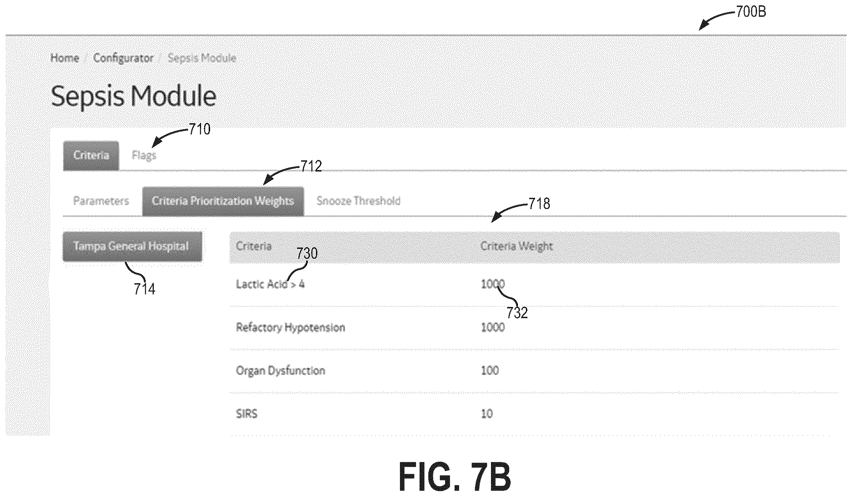

Turning to FIG. 7B, a second example of a configuration UI 700B is shown. Configuration UI 700B may be displayed by a display device viewable by a user (e.g., a facility administrator or care provider). While configuration UI 700A displays a parameters configuration view as the configuration type, configuration UI 700B displays criteria prioritization weights view as the configuration type. Configuration UI 700B includes the same configuration heading toggle 710, configuration type toggle 712, and facility name 714 as configuration UI 700A. In the criteria prioritization view displayed in configuration UI 700B, a configuration table 718 displays multiple columns and rows, including a criteria column and a criteria weight column. Each row includes a criteria and a corresponding weight. As an example, criteria 730 is "Lactic Acid >4," and a weight 732 assigned to criteria 730 is 1000. Similar to configuration table 716, an authorized user may adjust one or more of the elements/values herein described of configuration table 718 in order to configure the weight, and thus priority, of one or more sepsis criteria.

In some examples, the configuration module may allow an authorized user to configure a scope of a sepsis criteria or a parameter. For example, each criterion, such as SIRS or Organ Dysfunction, may be configured with a different scope. For example, SIRS may be configured with a scope of looking at any lab value or vital sign of a particular type within the most recent 6 hours while Organ Dysfunction may be configured with a scope of looking at the most recent lab value or vital sign of a particular type within the most recent twelve hours. In some examples, a parameter (e.g., a vital sign such as temperature) may also be configured with a scope, including looking at a lab value, vital sign, or other clinical indicator that corresponds to the parameter from a particular timeframe, such as the most recent value within the last 24 hours or other suitable timeframe. In this way, the user may be able to configure each criteria element to display different information, even if the lab values or vital signs looked at overlap.

The configuration module 228 may use the information from the configuration UI 700A and the configuration UI 700B, as well as other configuration UIs not depicted in the figures, in order to prioritize and weight criteria and define scopes and thresholds for criteria in order for the sepsis alert engine 202 to assign a patient a sepsis level based on patient data information. The configurable nature of the sepsis alert system 200 may allow for improved user interaction and therefore may allow the users to easier identify which of the sepsis level alerts provided by the system are valid or indicate treatment for a patient.

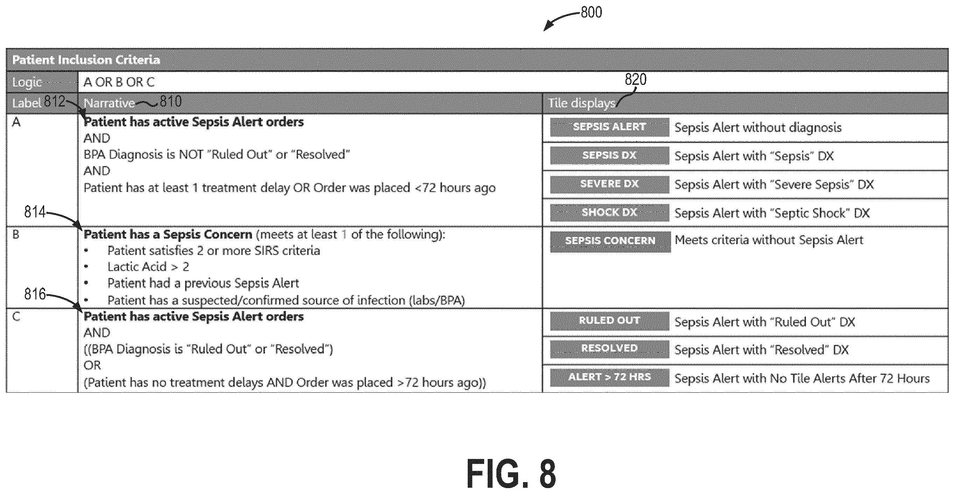

Referring now to FIG. 8, a table 800 of example patient inclusion criteria is shown. The table 800 includes the logic for which sepsis level may be assigned to and displayed as an element for a patient. For example, the sepsis alert engine 142 may apply the logic of table 800 in order to assign a sepsis level to each patient. The table includes a narrative column 810 and a tile displays column 820. The tile displays column 820 includes examples of sepsis level elements that correspond to sections of the narrative column 810. The narrative column 810 includes three sections: section A 812, section B 814, and section C 816. Section A 812 includes the narrative for when a patient has active sepsis alert orders in a connected EMR, best practice advisory (BPA) diagnosis is not ruled out or resolved, and the patient has at least one treatment delay or the sepsis alert order was placed less than 72 hours previously. A sepsis alert order may be placed by a user to an EMR system for a patient and if the order is active, the patient is currently undergoing treatment for sepsis. Section B 814 includes the narrative for when a patient has a "Sepsis Concern" (e.g., meets criteria for sepsis but does not have active sepsis alert orders) in the sepsis alert engine, which includes any of the following: the patient satisfies two or more SIRS criteria, the patient's lactic acid is greater than two, the patient had a previous sepsis alert, or the patient has a suspected/confirmed source of infection either from labs or BPA. Section C 816 includes the narrative for when a patient has active sepsis alert orders and either BPA diagnosis is "Ruled Out" or "Resolved" or the patient has no treatment delays and the sepsis alert" order was placed more than 72 hours previously.

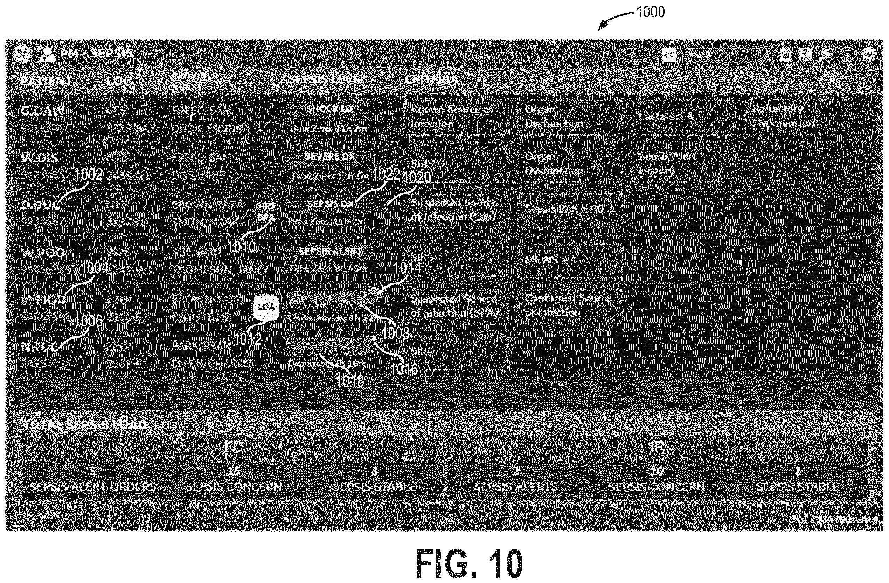

Different scenarios corresponding to the narratives described in each of the sections of the narrative column 810 may trigger display of different sepsis level elements in a GUI. For example, the Section A 812 narrative may correspond to "Sepsis Alert," "Sepsis Dx," "Severe Dx," or "Shock Dx" depending on particular patient parameters (and in particular based on the patient meeting the sepsis criteria as determined by the sepsis alert engine and based on a diagnosed sepsis level as determined from a user entry to the patient's EMR, for example). The Section B 814 narrative may correspond to "Sepsis Concern." Section C 816 may correspond to "Ruled Out," "Resolved," or "Alert > 72 HRS" depending on particular patient parameters and/or manual inputs from users. Other combinations of these or additional sepsis level elements are possible. The narratives herein described may be included in a rules module, such as rules module 214, and thus, the assigned sepsis level is based on rules applied in the sepsis alert engine. In some examples, a diagnosed sepsis level as input by a user (e.g., a clinician-based diagnosed entered into the patient's EMR) may also be included as a factor in the narrative for identifying a sepsis level. Alternatively, the sepsis alert engine may indicate a mismatch between the system-determined sepsis level, as based on the narrative described herein, and a diagnosed sepsis level as input by a user, as will be described later with reference to FIG. 10.

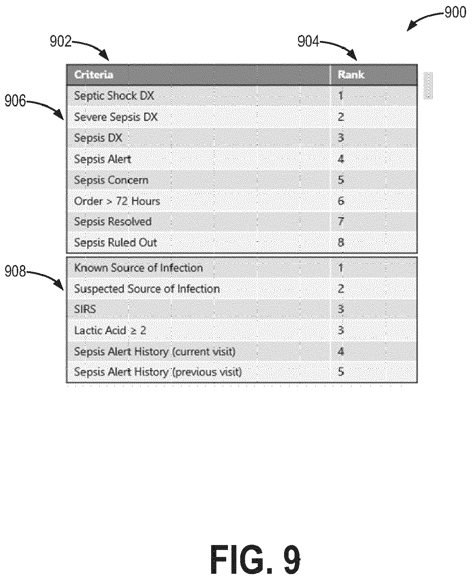

Turning now to FIG. 9, a table 900 of example sepsis prioritization is shown. The table 900 includes a criteria column 902 and a rank column 904. Three sections of rows comprise the table 900. A first section 906 comprises a prioritization of sepsis levels. A second section 908 comprises a prioritization of sepsis criteria.

In the example depicted in the table 900, the criteria column 902 with respect to the first section 906 includes rows of sepsis levels "Septic Shock DX," "Severe Sepsis DX," "Sepsis DX," "Sepsis Alert," "Sepsis Concern," "Order > 72 Hours," "Sepsis Resolved," and "Sepsis Ruled Out." In the example, criteria are given a priority rank from 1-8, wit 1 as the lowest number. For example, "Septic Shock DX" is given a rank of 1 in the rank column 904. Ranks with lower numbers are of increased priority and ranks with higher numbers are of decreased priority. In some examples, the different sepsis level elements that have different ranks may be displayed with different colors in a UI corresponding to their rank or to a set of ranks. For example, the sepsis level elements of rank 1, 2, 3, and 4 may be displayed in a UI with a red colored element, the sepsis level element of rank 5 may be displayed in the UI with an orange colored element, and the sepsis level elements of rank 6, 7, and 8 may be displayed in the UI with a gray colored element. Additional or alternative color displays are possible. Color-coding based on prioritization may aid a user to more easily identify a patient that may be imminently treated (or have treatment orders inputted) for sepsis.

In the example sepsis prioritization depicted in the table, the criteria column 902 with respect to the second section 908 includes rows of example sepsis criteria for a particular sepsis level element. Each sepsis criteria corresponds to a rank in the rank column 904. For example, criteria "Known Source of Infection", which may be one of the criteria that a patient meets in order to trigger a particular sepsis level, corresponds to the rank of 1 and "Sepsis Alert History (previous visit)" corresponds to the rank of 5. More than one criteria may have the same rank. Ranks with lower numbers (with 1 as the lowest number) are of increased priority, ranks with higher numbers are of decreased priority Sepsis criteria that are of increased priority may cause the sepsis alert engine to trigger a more severe sepsis level for a patient.

Turning now to FIG. 10, an example of a sepsis alert GUI 1000 is shown. Sepsis alert GUI 1000 may be displayed on a display device (e.g., a display device of a care provider device 134) viewable by a user (e.g., a care provider). Similar to sepsis alert GUI 300, sepsis alert GUI 1000 is depicted in command center view, showing multiple columns in a display including a patient information column, a patient location column, a sepsis level column, and a sepsis criteria column. Sepsis alert GUI 1000 additionally depicts display of flags and icons indicating active alerts for displayed patients.

As an example, a row corresponding to a first patient 1002 may include a flag 1010. Flag 1010 may indicate an active or dismissed SIRS BPA in a connected EMR. If active, the flag 1010 may be displayed with a color such as red. If dismissed or snoozed, the flag 1010 may be displayed with a color such as gray. The flag 1010 may be hovered over or selected (via a user input device like a mouse or trackpad connected to a care provider device such as care provider device 134) to display additional information in a pop-up UI, such as a comment or an acknowledged reason for a dismissal of the flag (if applicable). The pop-up UI may also include optional user inputs to dismiss or snooze the alert.

As another example, a row corresponding to a patient 1004 may include a flag 1012. Flag 1012 may indicate at least one active Line, Drain, Airway (LDA) order. The flag 1012 may be hovered over or selected (via a user input device like a mouse or trackpad connected to a care provider device such as care provider device 134) to display additional information in a pop-up LΠ. Such additional information may include a description and a placement date of an active or inactive (e.g., discontinued) LDA. More than one LDA may be displayed in the pop-up LΠ (if a patient has more than one LDA), including more than one active LDA and/or more than one inactive LDA, all with descriptions and placement dates. The description of an LDA may include characteristics of the LDA such as location, type, size, or other relevant characteristic.

As a further example, the row corresponding to first patient 1002 may also include a flag 1020. Flag 1020 may indicate a diagnosis mismatch. The sepsis alert GUI 1000 may display a diagnosis mismatch flag based on rules from the rules module 214. A diagnosis mismatch flag, such as flag 1020, indicates that a diagnosed sepsis level for a patient (e.g., a diagnosis manually input by a user which has been retrieved by the command center engine) does not match the system-determined sepsis level. A diagnosis mismatch flag may be displayed next to a first sepsis level element 1022 and may be displayed as either a red upward facing arrow, indicating that a patient meets criteria for a higher sepsis level, or a red downward facing arrow, indicating that a patient meets criteria for a lower sepsis level. The flag 1020 may be hovered over or selected to display additional information in an additional (e.g., pop-up) display panel of the current diagnosed sepsis level as well as information of the sepsis level matching the system-determined sepsis level.

Further, sepsis alert GUI 1000 may include one or more icons displayed within a sepsis level element. Icons may indicate that a sepsis level alert is under review or is snoozed. For example, icon 1014 is depicted within sepsis level element 1008 corresponding to a patient 1006. Icon 1014 is a review icon, indicating that the sepsis level element 1008 is under review by a user. As another example, icon 1016 is depicted within sepsis level element 1018. Icon 1016 is a snooze icon, indicating that the sepsis level alert displayed via sepsis level element 1018 is snoozed (e.g., dismissed). Icons, such as icon 1014 and icon 1016, may be removed from the display if the status of the sepsis level element changes, such as the review or snooze alert being canceled by a user or sepsis criteria being updated based on triggers from new patient data (e.g., newly acquired laboratory data or vital signs).

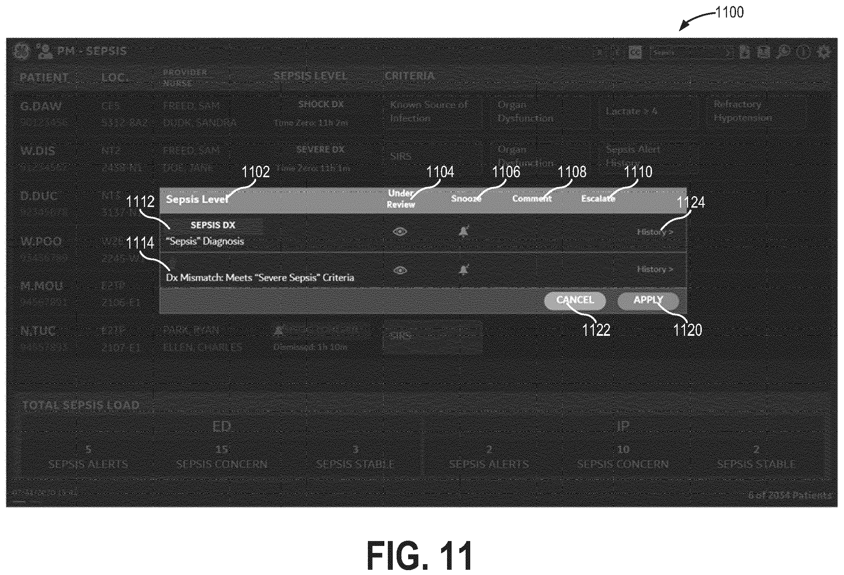

Referring now to FIG. 11, an example of a third pop-up LΠ 1100 is shown. Third pop-up LΠ 1100 may be displayed in response to hovering over or selecting a diagnosis mismatch flag (e.g., flag 1020 shown in FIG. 10)in a GUI, such as sepsis alert GUI 1000 and may display additional information relating to the flag that was selected. Additionally or alternatively, third pop-up UI 1100, or similar pop-up UIs that depict selectable elements to manually snooze or review alerts, may be displayed in response to hovering over or selecting a different element of a sepsis alert GUI. For example, a sepsis level element may be selected via a user input different than the user input used to launch first pop-up LΠ 500 depicted in FIG. 5 (e.g., first pop-up UI 500 may be launched via a left click input to a mouse and third pop-up UI 1100 may be launched via a right click input to a mouse, as a non-limiting example). Third pop-up UI 1100 includes a plurality of columns, including a sepsis level column 1102, an under review column 1104, a snooze column 1106, a comment column 1108, and an escalate column 1110, and a plurality of rows, including a sepsis level row 1112, and a diagnosis mismatch row 1114. In other similar pop-up UIs, additional or different rows and columns may be displayed in response to which element was selected on the sepsis alert GUI.

The sepsis level column 1102 includes a current sepsis level for a patient. The current sepsis level may be displayed as a sepsis level element on a GUI, such as sepsis alert GUI 1 000. The sepsis level column 1102 further includes a description of the diagnosis mismatch, including the sepsis level that the sepsis alert engine 202 calculates the patient to meet.

The under review column 1104 includes icons, corresponding to each row of the third pop-up UI 1100, that may be selected in order to display an icon on a UI (e.g., sepsis alert GUI 1000) to show that an alert is under review. For example, if an icon in the under review column 1104 corresponding to the sepsis level row 1112 were selected, an under review icon may be displayed within a corresponding sepsis level element in a GUI, such as sepsis alert GUI 1000. As another example, if an icon in the under review column 1104 corresponding to the diagnosis mismatch row 1114 were selected, an under review icon may be displayed within a diagnosis mismatch flag in a UI such as sepsis alert GUI 1000. Selecting an under review icon may display the under review icon to all users.

The snooze column 1106 includes icons, corresponding to each row of the third pop-up UI 1100, that may be selected in order to display an icon on a GUI (e.g., sepsis alert GUI 1000) to show that an alert is snoozed. For example, if an icon in the snooze column 1106 corresponding to the sepsis level row 1112 were selected, a snooze icon may be displayed within a corresponding sepsis level element in a GUI, such as sepsis alert GUI 1000. As another example, if an icon under the snooze column 1106 corresponding to the diagnosis mismatch row 1114 were selected, a snooze icon may be displayed within a corresponding diagnosis mismatch flag, such as flag 1020 of sepsis alert GUI 1000. Selecting a snooze icon may display the snooze icon to all users and also may snooze the corresponding alert it is attached to unless new patient data is acquired that unsnoozes the corresponding alert or a user manually unsnoozes the alert.

The comment column 1108 includes display areas for a user to optionally add a comment corresponding to one of the rows of the third pop-up UI 1100. For example, a user may add a comment to the sepsis level row 1112 that may be viewable by the user at a later time or by other users. The escalate column 1110 includes display areas for a user to optionally escalate an element to another user or group of users. For example, a user may escalate a sepsis level to another user by selecting or typing into the display area corresponding to the escalate column 1110 and the sepsis level row 1112.

Any action taken within the third pop-up UI 1100 may be applied to a sepsis alert GUI (e.g., sepsis alert GUI 1000) by selecting an apply button 1120. Any action taken within the third pop-up UI 1100 may be canceled by selecting a cancel button 1122, meaning that actions taken (e.g., selecting a snooze icon or an under review icon) will not be applied to a UI (e.g., sepsis alert GUI 1000).

Rows within third pop-up LΠ 1100may further display one or more history links that are selectable. For example, sepsis level row 1112 includes a history link 1124. Additional information may be displayed in response to selecting the history link 1124. Such additional information may include a selected patient's prior sepsis levels, review actions, or snooze actions which were stored in a history module of a sepsis alert engine (e.g., history module 224 of sepsis alert engine 202). For example, if a history link were selected, an additional display panel may be added which indicates that a patient to which the history link corresponds initially was assigned a "Sepsis Concern" level which was snoozed by a user and then the patient's sepsis level was upgraded to a "Sepsis Dx" level based on system-determined parameters, which unsnoozed the patient's sepsis level alert.

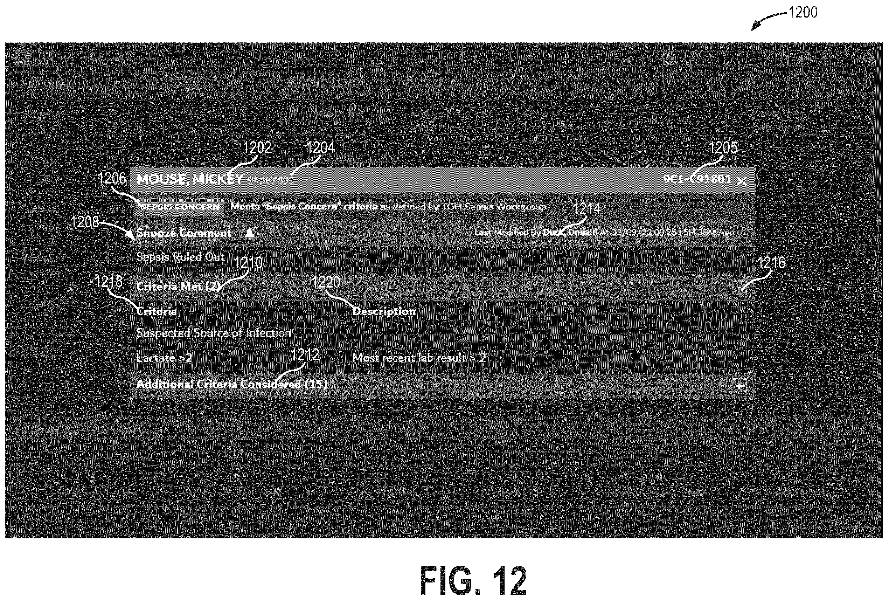

Referring now to FIG. 12, an example of a fourth pop-up LΠ 1200 is shown. Fourth pop-up UI 1200 may be displayed in response to hovering over or selecting a snoozed sepsis level element. Other snoozed alerts may be selectable to launch similar pop-up UIs with relevant additional information. For example, a snoozed sepsis level element, such as sepsis level element 1018 depicted in FIG. 10, may be selected or hovered over to display additional information in a pop-up UI, such as fourth pop-up UI 1200. Fourth pop-up UI 1200 includes a patient name 1202, an identification number 1204, and a patient location code 1205. Fourth pop-up UI 1200 may further include a sepsis level element 1206, a snooze comment 1208, a criteria met column 1210, an additional criteria considered column 1212, and a modification history description 1214.

The snooze comment 1208 may display a comment from a user describing the reason for snoozing the sepsis level, such as "ruled out" or "resolved". The modification history description 1214 may display the name of the user who most recently modified a snooze status for a corresponding sepsis level element along with a date and time of when the most recent modification occurred.

The criteria met column 1210 and the additional criteria considered column 1212 may be displayed similar to the criteria column 520 and additional criteria considered panel 536 described with respect to FIG. 5. Both the criteria met column 1210 and the additional criteria considered column 1212 may comprise a criteria column and a description column detailing criteria in the criteria column. For example, criteria met column 1210 contains criteria column 1218 which lists all of the criteria that a patient with patient name 1202 met and description column 1220 which lists descriptions corresponding to each criterion listed in the criteria column 1218. The criteria met column 1210 and the additional criteria considered column 1212 may be displayed in either an expanded view, which shows the criteria column and descriptions column, or in an unexpanded view, which does not show the criteria column and the descriptions column. An expansion button, such as expansion button 1216, may be selected to toggle between an unexpanded and an expanded view.

Turning now to FIG. 13, an example of a sepsis alert GUI 1300 is shown of a sepsis alert engine, such as the sepsis alert engine 202 of FIG. 2. Sepsis alert GUI 1300 may be similar to sepsis alert GUI 300 as described with regard to FIG. 3. Sepsis alert GUI 1300 may be displayed on a display device (e.g., a display device of a care provider device 134). Specifically, sepsis alert GUI 1300 may be displayed to a care provider when the care provider uses the sepsis alert engine to view patient information for one or more patients within a medical facility.

The sepsis alert GUI 1300 is displayed in a similar fashion as sepsis alert GUI 300 in that the sepsis alert GUI 300 displays information for one or more patients, including identifying information (e.g., a patient name, identification number, location code, and healthcare team) as well as a sepsis level element corresponding to a patient. While sepsis alert GUI 300, as depicted in FIG. 3, displays a column of sepsis criteria (e.g., sepsis criteria column 322), sepsis alert GUI 1300 alternatively displays a treatment alerts column 1302. Treatment alerts column 1302 displays treatment alerts corresponding to each displayed patient, such as "missing vasopressors," "missing fluids," "repeat lactate," or others, which may alert a user to treatment modalities that are indicated for a particular patient.

As an example, a patient 1306 with sepsis level element 1304 may have a treatment alert element 1308 that indicates to a user that the patient 1306 is missing fluids. The treatment alert element 1308 may be hovered over or selected in order to display additional information in a pop-up LΠ regarding the treatment alert referenced in treatment alert element 1308. Additionally, the pop-up UI may display options for updating a status of the treatment alert, such as changing the status from incomplete to complete or from due to ordered. Once a treatment alert has been completed or dismissed, it may no longer appear in the sepsis alert GUI 1300.

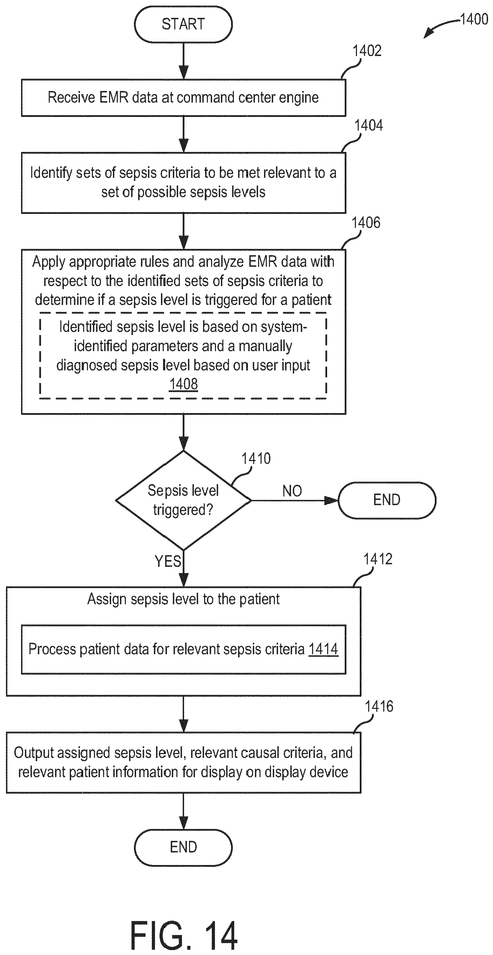

Turning now to FIG. 14, a flowchart illustrating a method 1400 for triggering and displaying a sepsis level alert is shown. Method 1400 may be executed according to instructions stored in memory of a sepsis alert system of a computing device and executed by one or more processors of the computing device, such as computing device 102 of FIG. 1. As explained above, the computing device 102 may include a command center engine 140 that receives signals from one or more EMR databases (e.g., EMR database 280), analyzes the signals, and triggers alerts via sepsis alert engine 142 and or sepsis alert engine 202, which are then displayed via an appropriate GUI, such as sepsis alert GUI 300, sepsis alert GUI 400, sepsis alert GUI 1000, and/or sepsis alert GUI 1300.

At 1402, the method 1400 includes receiving EMR data at the command center engine. The EMR data may include information about one or more patients being treated at a specific medical facility or medical network. The EMR data may include information regarding patient vital signs, lab values, algorithmic scores, provider orders (medications, fluids, lab orders, imaging orders, etc.), diagnostic imaging exams, procedures, historical data (prior hospitalization, prior sepsis diagnoses or sepsis alert orders), and so forth.

At 1404, method 1400 includes identifying a set of sepsis criteria relevant to a set of possible sepsis levels. In some examples, the set of sepsis criteria may be based on configured parameters or scopes for a particular sepsis criteria or sepsis level. An authorized user may input values/elements in order to configure thresholds, scope, etc. for parameters and sepsis criteria, as described with reference to FIGS. 7A and 7B. Additionally, each sepsis level may include a different set of sepsis criteria that may be met in order to trigger a particular sepsis level. Additionally, in some examples, a sepsis criterion may include a set of parameters therein that may be met in order to meet the sepsis criterion, as is with a SIRS criterion.

At 1406, method 1400 includes applying appropriate rules to and analyzing the received EMR data with respect to the sets of criteria identified at 1404 to determine if a sepsis level has been triggered for a patient. As explained above with respect to FIG. 2, the sepsis alert engine may include a rules module that applies rules to the received EMR data to determine if the EMR data have triggered any alerts (e.g., sepsis levels) for one or more patients. Each alert may relate to a sepsis level of a specific priority and the EMR data may include causal criteria (e.g., sepsis criteria) or parameters that trigger a sepsis level for a patient. Logic or narratives may be applied to the data retrieved from the one or more EMR systems in order to identify a sepsis level to be assigned to a patient, as described with reference to FIG. 8. The sepsis level that is identified or triggered may be based on both the system-identified patient parameters identified within the EMR data as well as a manually diagnosed sepsis level as indicated based on user input to an EMR, as indicated at 1408. Analysis of EMR data may additionally include determining a mismatch between a system-identified sepsis level and a manually diagnosed sepsis level.

At 1410, method 1400 judges whether a sepsis level has been triggered for a patient. Once the EMR data has been retrieved, the sets of criteria available to be met for the set of possible sepsis levels have been identified, the EMR data has been analyzed based on applied appropriate rules from the rules module, the command center engine (or sepsis alert engine) may determine whether a sepsis level has been triggered for the patient. If a sepsis level is triggered for the patient, method 1400 proceeds to 1412. If a sepsis level is not triggered for the patient, method 1400 ends. It should be appreciated that when a sepsis level is not triggered for a patient, that patient may still be listed on the sepsis alert GUI, but without a sepsis level element. Alternatively, when a sepsis level is not triggered for the patient, that patient may not be listed on the sepsis level GUI.

At 1412, method 1400 includes assigning a sepsis level to the patient. The command center engine, using the above described process may trigger a sepsis level for the patient. The sepsis level may then be assigned to the patient within the sepsis alert engine. At 1414, method 1400 includes processing patient data for the sepsis criteria relevant to the assigned sepsis level. Once the command center engine has determined which sepsis criteria have been met for the assigned sepsis level, the command center engine may proceed to process all patient data relevant to the assigned sepsis level from one or more EMR systems and/or other data repositories of the hospital system, including sorting sepsis criteria relevant to the assigned sepsis level into categories, including a first category of criteria that the patient did meet and a second category of the criteria that the patient did not meet. The first and second categories may be displayed in modified sepsis alert GUIs or pop-up UIs to indicate sepsis criteria and parameters that a patient met and did not meet.

At 1416, method 1400 includes outputting the assigned sepsis level, relevant causal criteria, and relevant patient information for display on a display device. For example, as described above in reference to FIG. 3, a sepsis alert GUI may display an assigned sepsis level for a patient alongside one or more sepsis criteria that triggered the assigned sepsis level for that patient in a display panel of the sepsis alert GUI. Additional display elements may be displayed on the sepsis alert GUI, as is described below.

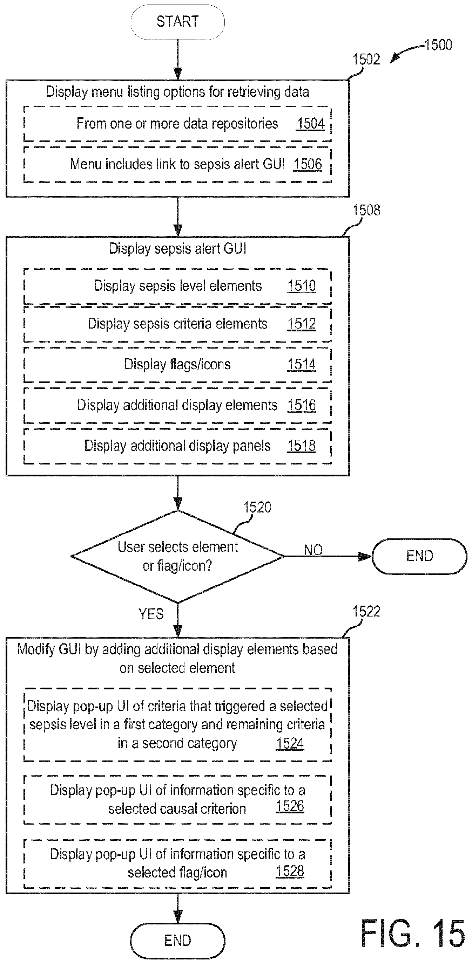

Referring now to FIG. 15, a flowchart illustrating a method 1500 for viewing and modifying a sepsis alert GUI is shown. Method 1500 may be implemented by the sepsis alert system in communication with one or more additional data repositories (e.g., computer systems and databases), such as one or more EMR systems. In some examples, aspects of method 1500 may be carried out by a sepsis alert system, such as sepsis alert system 200 of FIG. 2, according to instructions stored in a memory (e.g., non-transitory memory 106) of the sepsis alert system, which may be executed by one or more processors (e.g., processor 104) of the sepsis alert system.

At 1502, method 1500 includes displaying a menu listing options for retrieving data. The data may include data of one or more patients and may be obtained from a plurality of databases and systems of a hospital. As indicated at 1504, the options listed in the menu may include an option to retrieve data from one or more data repositories such as an EMR system. Further, the options listed in the menu may include an option to display a sepsis alert graphical user interface (GUI), and thus the menu may include a link to the sepsis alert GUI, as indicated at 1506. In some examples, the sepsis alert GUI may be accessed from the one or more data repositories. For example a user may launch an EMR interface whereby one or more EMRs of a patient may be viewed. The EMR interface may include a link to the sepsis alert GUI that, when selected, launches the sepsis alert GUI, as indicated at 1506. In some examples, the menu may include a command center or hospital systems menu whereby the sepsis alert GUI and the EMR interface may be accessed via separate menu options.

At 1508, method 1500 includes displaying the sepsis alert GUI. As explained previously, the sepsis alert GUI may be displayed in response to selection of a link, which may be displayed via the EMR interface, a command center interface, a hospital systems menu, or other suitable interface. The sepsis alert GUI may display a respective sepsis level element for one or more patients, as indicated at 1510. The sepsis levels may be generated as explained above with respect to FIG. 14, and the sepsis levels may be displayed in the sepsis alert GUI as interface elements, such as the sepsis level element 330 of FIG. 3. The sepsis alert GUI may be displayed on one or more suitable display devices, such as a display of a care provider device.

The sepsis alert GUI may also display one or more sepsis criteria elements corresponding to the sepsis level for a specified patient, as indicated at 1512. The sepsis criteria may be determined, identified, or generated as explained above with respect to FIG. 14, and the sepsis criteria may be displayed in the sepsis alert GUI as interface elements, such as sepsis criteria element 332 of FIG. 3. Sepsis level elements, such as sepsis level element 330 of FIG. 3, may be color coded based on the priority rank of the sepsis level, as described with respect to FIG. 9. Additionally, an order in which patients are displayed on the sepsis alert GUI may be based on the priority rank of each patient's assigned sepsis level. Alternatively, patients may be displayed on the sepsis alert GUI in an order based on a chosen filter, as described with reference to FIG. 3.

In some examples, the sepsis alert GUI may display one or more flags and/or one or more icons for one or more patients, as indicated at 1514. The flags or icons may indicate patient information, such as an LDA flag (e.g., flag 1012), a sepsis diagnosis mismatch, such as a diagnosis mismatch flag (e.g., flag 1020), that an element is snoozed, such as a snooze icon (e.g., icon 1016), that an element is under review, such as an under review icon (e.g., icon 1014), or a best practice advisory for a sepsis criteria (e.g., flag 1010).

In some examples, the sepsis alert GUI may display additional elements, as indicated at 1516. In some examples, a sepsis level element or sepsis criteria element may be selectable to modify the sepsis alert GUI by adding additional display elements to the sepsis alert GUI and the additional display elements may identify criteria or parameters from the retrieved data from the one or more data repositories that triggered the displayed sepsis level or sepsis criteria. For example, a sepsis level element displayed on the sepsis alert GUI may be selectable to launch a pop-up UI, such as first pop-up LΠ 500 of FIG. 5. Additionally or alternatively, a flag or icon may be selectable to modify the sepsis alert GUI by adding additional display elements to the sepsis alert GUI identifying information specific to the selected flag or icon. Thus, as indicated at 1518, additional display panels may be displayed via the sepsis alert GUI in response to selection of a sepsis level element, sepsis criteria element, flag, icon, or other user interface element. For example, a sepsis level element may be selectable to launch a first display panel with additional information and/or controls relating to the associated sepsis level (e.g., first pop-up LΠ 500 of FIG. 5), and the sepsis alert GUI may further include a limited set of additional visual elements that are selectable to launch a second display panel. The limited set of additional visual elements may include, for each of one or more patients, one or more causal criteria each including a criterion of the plurality of criteria that triggered the sepsis level. For example, the limited set of visual elements may be the sepsis criteria elements described above with respect to FIG. 3. If one or more visual elements is selected, a pop-up UI, such as second pop-up UI 600, may be displayed. In this way, the second display panel may include one or more values of a selected causal criterion, the one or more values obtained from the retrieved data (e.g., from the EMR systems).

At 1520, method 1500 determines if a user has selected an element, flag, or icon. The user (which may be a care provider) may enter a user input to an element, flag, or icon, such as a mouse or touch pad hover, or a mouse click or touch pad input, in order to view additional information associated with the element, flag, or icon. If the user has selected an element, flag, or icon, method 1500 proceeds to 1522 to display additional information based on the input.

At 1522, method 1500 includes displaying the additional information based on which element was selected. The additional information may include, as indicated at 1524, displaying a pop-up UI of criteria that triggered a selected sepsis level element in a first category and remaining criteria in a second category, such as first pop-up UI 500. In some examples, displaying the additional information may include, as indicated at 1526, displaying a pop-up LΠ of information specific to a selected sepsis criteria element, such as second pop-up UI 600. In other examples, displaying the additional information may include, as indicated at 1528, displaying a pop-up LΠ of information specific to a flag or icon, such as third pop-up UI 1100 or fourth pop-up UI 1200. The patient specific information displayed in any of the optional pop-up UIs described herein may be obtained from the patient's EMR stored within one or more data repositories (e.g., one or more EMR systems).

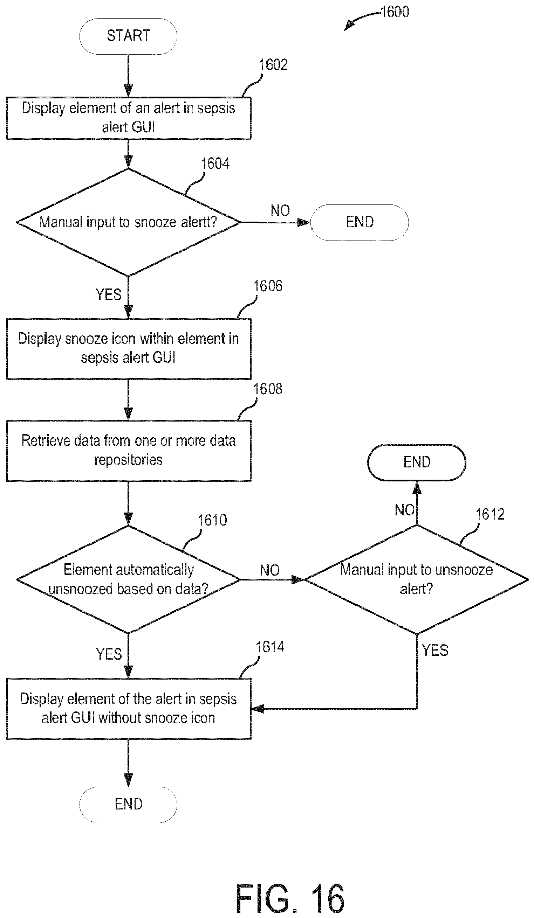

Referring now to FIG. 16, a flowchart illustrating a method for snoozing and/or unsnoozing elements within a sepsis alert GUI is shown. Method 1600 may be implemented by the sepsis alert system in communication with one or more additional data repositories (e.g., computer systems and databases), such as one or more EMR systems. In some examples, aspects of method 1600 may be carried out by a sepsis alert system, such as sepsis alert system 200 of FIG. 2, according to instructions stored in a memory (e.g., non-transitory memory 106) of the sepsis alert system, which may be executed by one or more processors (e.g., processor 104) of the sepsis alert system.

At 1602, method 1600 includes displaying one or more elements, such as a sepsis level element, a sepsis criteria element, a flag, or an icon, in a sepsis alert GUI, such as sepsis alert GUI 1000. Such elements may indicate an alert within a sepsis alert system, such as sepsis alert system 200 described in reference to FIG. 2. The elements may be selectable, as previously described, to add additional display elements in a pop-up UI.

At 1604, method 1600 judges whether a user has manually snoozed an alert. The user (who may be a care provider) may enter a user input to an element, flag, or icon, such as a mouse or touch pad hover, or a mouse click or touch pad input, in order to snooze an alert. For example, a user may define an alert for a given patient as snoozed via interaction with a sepsis alert GUI or an additional display panel (e.g., a pop-up UI). If the alert has been manually snoozed, method 1600 proceeds to 1606. If the element has not been manually snoozed, method 1600 ends.

At 1606, method 1600 includes adding a snooze icon to the element, indicating that the alert was manually snoozed. The snooze icon may be displayed in the sepsis alert GUI in which the snoozed element is displayed. The snooze icon may be displayed within the element in order to indicate that the alert is snoozed. In some examples, when an alert is snoozed, the color of the corresponding element may be changed, for example changed to gray, in order to indicate visually to a user (e.g., a care provider) that the element is currently snoozed.

At 1608, method 1600 includes retrieving data from one or more data repositories, such as one or more EMR systems. Data retrieval may occur in real time or at regular, defined, intervals as previously discussed. The element that is snoozed may have been triggered based on data from a first retrieval time (e.g., lab values, vital signs, etc. from a first time). New data for the patient may be acquired at a second time (e.g., updated lab values, repeated vital signs, etc.). Depending on the new data for the patient, the patient may meet additional parameters or sepsis criteria at the second time that they did not meet at the first time. Method 1600 proceeds to 1610.

At 1610, method 1600 judges if the snoozed alert is automatically unsnoozed based on data retrieved at 1608. If the patient meets additional parameters or sepsis criteria at the second time (or other later times) that data is retrieved, the alert that is snoozed may be automatically unsnoozed in the event that there is additional criteria or parameters met for the alert or in the event that the alert is changed based on the additional criteria or parameters. Additionally or alternatively, an authorized user may change the configured thresholds, scope, etc. of one or more parameters or sepsis criteria between the first and second data retrieval times and, thus, patient data may indicate a patient meets one or more parameters at the second time because the threshold, scope, etc. is different, even if the patient data has not changed between the first and second times.

As an example, if the alert that was snoozed was a sepsis level indicating that the patient in question met criteria for a "Sepsis Dx," but additional parameters met at the second time based on additional data retrieved may trigger the patient for a sepsis level of "Septic Shock," the alert may be automatically unsnoozed in order to indicate to a user that the patient now meets criteria for a more severe sepsis level. In other examples, new criteria or parameters may be met with respect to the alert that is snoozed, but the element may not be changed to a different element. In such cases when the alert is not changed to a different alert (e.g., "Sepsis Dx" changed to "Septic Shock"), the alert may still be automatically unsnoozed in order to indicate to a user that additional criteria are now available to be reviewed that were not present at the time that the alert was snoozed initially. If the alert is automatically unsnoozed based on any of the above described reasons, the method 1600 proceeds to 1614. If not, the method 1600 proceeds to 1612.

At 1612, method 1600 judges if whether the snoozed alert has been unsnoozed based on manual input from a user. Similar to how a user may manually snooze an alert, as described at 1604, a user may also manually unsnooze an alert. For example, a user may define the snoozed alert for the patient as unsnoozed via interaction with the sepsis alert GUI or an additional display panel (e.g., a pop-up UI). If the alert has been manually unsnoozed, method 1600 proceeds to 1614. If the element has not been manually unsnoozed, method 1600 ends.

At 1614, method 1600 includes displaying the element of the alert that has been unsnoozed without the snooze icon that was present in the sepsis alert GUI when the alert was snoozed. In some examples, removal of a snooze icon (e.g., unsnoozing the element) may produce an alert to the user or users indicating that the alert has been automatically unsnoozed based on new data or that the alert has been manually unsnoozed by another user (e.g., if a first user manually unsnoozes the alert, a second user may receive an alert in the sepsis alert GUI indicating that the element for that patient is no longer snoozed). If the alert, when unsnoozed, is displayed via an element of a particular color but that color is changed (to gray, for example) when the alert is snoozed, the original color of the element may be restored when the snooze icon is removed when the alert is unsnoozed. Alternatively, the color may be changed to a different color in the event the sepsis level (or other alert) has changed to an alert configured to display via an element of a different color.

When the sepsis alert GUI is displayed and/or when the additional display elements (e.g., the limited set of visual elements and/or the various pop-up UIs described herein) are displayed, the one or more EMRs may exist in an un-launched state (e.g., not displayed and not currently access by the display device.) In this way, the data from the EMRs may be used to generate the sepsis alerts and values of causal criteria may be obtained from the EMRs (e.g., lab values, vital signs, algorithmic scores, etc.) and used to populate additional display panels without the user having to access the EMRs themselves (e.g., in a separate window). In doing so, the sepsis alert GUI as disclosed herein may present a limited set of information (e.g., sepsis levels and causal criteria triggering the sepsis levels) to the users/care providers in order to increase efficiency of the users' interaction with the available data, as users are not forced to sift through multiple separate EMRs, data feeds, data files, etc., to identify and then aggregate the needed information.

The systems, methods, and graphical user interfaces provided herein may improve the accuracy and timeliness of sepsis treatments and patient monitoring for sepsis out of high-demand, high-resource hospital units such as ICUs and EDs, which may be particularly useful during high-demand situations where time is limited, such as during infectious disease outbreaks where patient demand of resources may change exponentially and data obtained one day may not be applicable the next day. The time it would take to individually collect data from multiple EMRs, aggregate and analyze the collected data, and visualize the data using standard methods could render the data useless by the time the data was visualized, because patient conditions may change so rapidly. By establishing a system that automatically receives data from all EMRs in real time or near real time, aggregates that information regardless of data format, and continually updates a sepsis alert GUI as data is received, the approach of the disclosure allows care providers to make informed decisions about sepsis treatments, such as when to administer antibiotics or fluids and when to continue monitoring without intervention, thereby improving patient care.

In contrast, in prior systems when a care provider attempted to determine if a patient met criteria for a sepsis diagnosis, errors and/or delays could be made due to delays in individual care providers obtaining all necessary parameters for evaluating a patient's condition. For example, a patient's condition may have changed to the point where the patient requires different treatment interventions, but limited care provider resources may result in the patient's treatment being delayed. The described sepsis alert GUI addresses this problem by providing a specific, dynamically updating list of patient sepsis levels and associated causal/triggering criteria. In this way, the sepsis alert GUI provides an improvement to the capability of the healthcare system as a whole. The disclosure provides a specific way of improving the capability of the healthcare system, by providing one or more sepsis alert GUIs that display dynamically updating patient sepsis level lists. The disclosure further provides a specific improvement to the way computers operate by aggregating EMR data for multiple patients in one location and updating the patient sepsis level in real time, which may obviate the need for users to have to navigate through multiple different EMRs/data files, manually update information as patient status changes, and so forth, thereby increasing the efficiency of the operation of the computer for the user.

The sepsis alert GUIs described herein provide a specific manner of displaying a limited set of information to a user (patient sepsis level information and associated causal criteria), rather than using conventional user interface methods to display a generic index on a computer, requiring the user to step through many layers of menu options to access the desired data, or burying the desired data within all hospital data. Thus, the user experience with the computer may be improved and made more efficient.

Furthermore, by displaying a limited set of information via the sepsis alert GUIs as described herein, operation of the computing device(s) that collect and render the data for display may be improved by reducing the processing demands of the computing device(s), thereby increasing the efficiency of the computing device(s). For example, only certain causal criteria leading to a specific sepsis level may be displayed, which results in a limited amount of the data that is received being processed, which may improve the efficiency of the computing device(s). Further, in some examples, the data is processed in real time and updates to the sepsis alert GUIs are made continuously as data is received, and therefore undue processing lags that occur if the updates were made at predefined time points may be reduced, which may improve the efficiency of the computing device(s). Additionally, as described herein, if network lags or outages result in an incomplete sepsis alert (e.g., due to one or more criteria for evaluating sepsis level not being currently available), users may be notified that a sepsis alert is not available rather than being presented with an incomplete and hence potentially inaccurate sepsis alert.

The sepsis alert system, included as a part of a medical information system as described herein, may provide for high speed data processing that allows for the real time or near real time updates to the sepsis alert GUIs. For example, the sepsis alert system may be configured to update the sepsis alert GUIs every 30 seconds, rather than every 15 minutes or longer as in some prior systems.

Thus, via the disclosed sepsis alert system, patient sepsis levels and information relevant to a patient status may be displayed in a manner that is easy to visually parse and act on in a reduced amount of time. The patient information may be displayed via small graphical elements with minimal text, which may allow a large number of sepsis levels to be included on the same screen. A user may then select a graphical element of interest to view more information about a corresponding event, record, or report. The patient information may be stored in different data repositories that would otherwise be accessed via individual interfaces, and by using the sepsis alert system to aggregate the patient information, an amount of time necessary to review relevant patient information for diagnosis and treatment decisions may be reduced. The sepsis alert system disclosed herein may also aggregate patient data to a single place, into a single application, which may help to reduce wasted time spent searching for known but scattered data, and unknown and missing data. This may reduce cognitive overloads and aid clinical thinking; because the patient data is reconstructed into a clinically helpful structure. The technical effect of automatically generating sepsis alerts (e.g., sepsis levels, sepsis criteria, flags, etc.) for a hospital unit such as an ICU or ED by applying a set of rules to selected patient data is that errors or delays due to sorting through large amount of patient data may be reduced, and an amount of time spent by a clinician making sepsis diagnoses may be reduced.

The disclosure also provides support for a computing device comprising a display screen, the computing device being configured to display on the display screen a menu listing one or more electronic medical records (EMRs) of one or more patients, and additionally being configured to display on the display screen a sepsis alert graphical user interface (GUI) accessible from the menu, wherein the sepsis alert GUI displays, for each patient, a sepsis level element identifying a sepsis level for that patient and a limited list of criteria that triggered the identified sepsis level, the limited list of criteria obtained from the one or more EMRs, each criterion in the limited list being selectable to launch a pop-up UI with additional information relating to the identified sepsis level and enable at least the selected criterion to be seen within the pop-up UI, and wherein the sepsis alert GUI is displayed while the one or more EMR systems are in an un-launched state. In a first example of the system, the sepsis level element is selectable to launch a second pop-up LΠ with additional information for each criterion of the limited list of criterion and remaining criteria that did not trigger the identified sepsis level. In a second example of the system, optionally including the first example, the limited list of criteria includes a systemic inflammatory response syndrome (SIRS) criterion, and wherein the SIRS criterion is selectable to launch a SIRS pop-up UI that enables a plurality of patient parameters that triggered the SIRS criterion to be displayed within the SIRS pop-up UI. In a third example of the system, optionally including one or both of the first and second examples, the identified sepsis level is assigned from a set of possible sepsis levels based on patient parameters received from the one or more EMR systems, the patient parameters including a diagnosed sepsis level, and wherein the limited list of criteria comprises a subset of the patient parameters that triggered assignment of the identified sepsis level. In a fourth example of the system, optionally including one or more or each of the first through third examples, a first sepsis level element for a first patient includes a mismatch flag indicating that the diagnosed sepsis level for the first patient does not match a system-determined sepsis level determined based on patient parameters for the first patient received from the one or more EMR systems. In a fifth example of the system, optionally including one or more or each of the first through fourth examples, a first sepsis level element for a first patient includes a snooze icon indicating that an identified sepsis level of the first patient has been snoozed. In a sixth example of the system, optionally including one or more or each of the first through fifth examples, the snooze icon is removed responsive to a determination that the identified sepsis level for the first patient has changed, the determination based on patient parameters received from the one or more EMR systems.

The disclosure also provides support for a method for a sepsis alert system, comprising: displaying a menu listing one or more options for retrieving data of one or more patients from a plurality of data repositories of a hospital, the plurality of data repositories including one or more electronic medical record (EMR) systems, and displaying a sepsis alert graphical user interface (GUI) that displays, for each patient, a sepsis level element indicating an assigned sepsis level determined from the retrieved data from the one or more EMR systems, in response to a selection of the sepsis level element, adding additional display elements to the sepsis alert GUI, the additional display elements identifying parameters from the retrieved data from the one or more EMR systems that triggered the assigned sepsis level, wherein the sepsis alert GUI is displayed while the one or more EMR systems are in an un-launched state. In a first example of the method, the assigned sepsis level is assigned based on a set of rules applied to the retrieved data, the set of rules identifying a plurality of criteria to be met in order to assign the assigned sepsis level. In a second example of the method, optionally including the first example, the identified parameters of the additional display elements comprise one or more causal criteria of the assigned sepsis level, the one or more causal criteria including one or more criteria of the plurality of criteria that triggered the assigned sepsis level. In a third example of the method, optionally including one or both of the first and second examples, the additional display elements include the identified parameters displayed in a first category and further include remaining criteria of the plurality of criteria displayed in a second category. In a fourth example of the method, optionally including one or more or each of the first through third examples, the sepsis alert GUI includes, for a first patient, a criteria element listing a causal criterion for the assigned sepsis level for the first patient, the causal criterion including a criterion of the plurality of criteria that triggered the assigned sepsis level for the first patient. In a fifth example of the method, optionally including one or more or each of the first through fourth examples, the criteria element is selectable to add a second additional display element to the sepsis alert GUI, the second additional display element including information specific to the causal criterion obtained from the retrieved data from the one or more EMR systems.

The disclosure also provides support for a sepsis alert system, comprising: one or more processors, and memory storing instructions executable by the one or more processors to: receive, at the sepsis alert system, a feed from an electronic medical record database, where one or more values for each of a plurality of parameters for each of a plurality of patients are sent over the feed, output, for display on a display device, a sepsis alert graphical user interface (GUI) that includes, for one or more patients of the plurality of patients, a sepsis level element indicating an assigned sepsis level for that patient, where each sepsis level element is generated by applying a set of rules to a set of patient data obtained from the feed, and display, on the sepsis alert GUI, for a selected patient of the one or more patients, a criteria element indicating a contributing factor to an assigned sepsis level of the selected patient. In a first example of the system, the assigned sepsis level for the selected patient is based in part on a diagnosed sepsis level for the selected patient, and wherein the instructions are executable to display, on the sepsis alert GUI, a mismatch flag for the selected patient responsive to the diagnosed sepsis level for the selected patient not matching a system-determined sepsis level for the selected patient, the system-determined sepsis level determined by applying the set of rules to a subset of the patient data obtained from the feed specific to the selected patient. In a second example of the system, optionally including the first example, the instructions are executable to display, on the sepsis alert GUI, a snooze icon indicating the assigned sepsis level of the selected patient has been snoozed by a user. In a third example of the system, optionally including one or both of the first and second examples, the instructions are executable to continue to apply the set of rules to a subset of the patient data obtained from the feed specific to the selected patient while the assigned sepsis level is snoozed and remove the snooze icon responsive to a determination that the snooze should be unsnoozed and/or the assigned sepsis level has changed based on application of the set of rules to the subset of the patient data. In a fourth example of the system, optionally including one or more or each of the first through third examples, the instructions are executable to determine that the snooze should be unsnoozed and/or the assigned sepsis level has changed based on a change to the set of rules and/or a change in one or more parameters of the subset of the patient data. In a fifth example of the system, optionally including one or more or each of the first through fourth examples, the assigned sepsis level for that patient is selected from a set of possible sepsis levels including sepsis alert, sepsis diagnosis, severe sepsis diagnosis, septic shock diagnosis, sepsis concern, sepsis ruled out, sepsis resolved, and no sepsis alert for a duration. In a sixth example of the system, optionally including one or more or each of the first through fifth examples, the criteria element is selectable to launch a pop-up LΠ including additional information of the contributing factor to the assigned sepsis level of the selected patient.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A computing device (102) comprising a display screen (122), the computing device (102) being configured to display on the display screen (122) a menu listing one or more electronic medical records (EMRs) (280) of one or more patients, and additionally being configured to display on the display screen (122) a sepsis alert graphical user interface (GUI) (300) accessible from the menu, wherein the sepsis alert GUI (300) displays, for each patient, a sepsis level element (330) identifying a sepsis level for that patient and a limited list of criteria (322) that triggered the identified sepsis level, the limited list of criteria (322) obtained from the one or more EMRs (280), each criterion in the limited list being selectable to launch a pop-up LΠ (600) with additional information relating to the identified sepsis level and enable at least the selected criterion to be seen within the pop-up UI (600), and wherein the sepsis alert GUI (300) is displayed while the one or more EMRs (280) are in an un-launched state.

2. The computing device (102) of claim 1, wherein the sepsis level element (330) is selectable to launch a second pop-up UI (500) with additional information for each criterion of the limited list of criterion (518) and remaining criteria (536) that did not trigger the identified sepsis level.

3. The computing device (102) of claim 1, wherein the limited list of criteria includes a systemic inflammatory response syndrome (SIRS) criterion, and wherein the SIRS criterion is selectable to launch a SIRS pop-up UI (600) that enables a plurality of patient parameters that triggered the SIRS criterion to be displayed within the SIRS pop-up LΠ (600).

4. The computing device (102) of claim 1, wherein the identified sepsis level is assigned from a set of possible sepsis levels based on patient parameters received from the one or more EMRs (280), the patient parameters including a diagnosed sepsis level, and wherein the limited list of criteria comprises a subset of the patient parameters that triggered assignment of the identified sepsis level.

5. The computing device (102) of claim 4, wherein a first sepsis level element (1022) for a first patient (1002) includes a mismatch flag (1020) indicating that the diagnosed sepsis level for the first patient (1002) does not match a system-determined sepsis level determined based on patient parameters for the first patient (1002) received from the one or more EMRs (280).

6. The computing device of claim 2, wherein a first sepsis level element (1018) for a first patient (1006) includes a snooze icon (1016) indicating that an identified sepsis level of the first patient has been snoozed.

7. The computing device of claim 6, wherein the snooze icon (1016) is removed responsive to a determination that the identified sepsis level for the first patient has changed, the determination based on patient parameters received from the one or more EMRs (280).

8. The computing device (102) of claim 5, wherein the identified sepsis level for a patient is based in part on the diagnosed sepsis level for the patient, and wherein computer readable instructions in a memory (106) are executable to display, on the sepsis alert GUI (300), the mismatch flag (1020) for the patient responsive to the diagnosed sepsis level for the patient not matching a system-determined sepsis level for the patient, the system-determined sepsis level determined by applying the set of rules to a subset of the patient data obtained from the one or more EMRs (280) specific to the patient.

9. The computing device (102) of claim 6, wherein computer readable instructions stored in memory (106) are executable to display, on the sepsis alert GUI (300), the snooze icon (1016) indicating the identified sepsis level of the patient has been snoozed by a user and to continue to apply the set of rules to a subset of the patient data obtained from one or more EMRs (280) specific to the patient while the identified sepsis level is snoozed and remove the snooze icon (1016) responsive to a determination that the snooze should be unsnoozed and/or the identified sepsis level has changed based on application of the set of rules to the subset of the patient data.

10. A method for a sepsis alert system, comprising:
displaying a menu (1502) listing one or more options for retrieving data of one or more patients from a plurality of data repositories of a hospital, the plurality of data repositories including one or more electronic medical record (EMR) systems (1504), and
displaying a sepsis alert graphical user interface (GUI) (1508) that displays, for each patient, a sepsis level element (1510) indicating an assigned sepsis level determined from the retrieved data from the one or more EMR systems (280);
in response to a selection of the sepsis level element (1520), adding additional display elements (1522) to the sepsis alert GUI, the additional display elements identifying parameters from the retrieved data from the one or more EMR systems that triggered the assigned sepsis level (1524), wherein the sepsis alert GUI is displayed while the one or more EMR systems are in an un-launched state.

11. The method of claim 10, wherein the assigned sepsis level is assigned based on a set of rules applied to the retrieved data (1406), the set of rules identifying a plurality of criteria to be met in order to assign the assigned sepsis level.

12. The method of claim 11, wherein the identified parameters of the additional display elements comprise one or more causal criteria of the assigned sepsis level (1404), the one or more causal criteria including one or more criteria of the plurality of criteria that triggered the assigned sepsis level.

13. The method of claim 12, wherein the additional display elements include the identified parameters displayed in a first category and further include remaining criteria of the plurality of criteria displayed in a second category (1416).

14. The method of claim 11, wherein the sepsis alert GUI includes, for a first patient, a criteria element listing a causal criterion for the assigned sepsis level for the first patient (1512), the causal criterion including a criterion of the plurality of criteria that triggered the assigned sepsis level for the first patient.

15. The method of claim 14, wherein the criteria element is selectable (1520) to add a second additional display element to the sepsis alert GUI, the second additional display element including information specific to the causal criterion (1526) obtained from the retrieved data from the one or more EMR systems.
